# EUROPEAN PATENT SPECIFICATION

(11) **EP 4 439 002 B1**
(45) Date of publication and mention of the grant of the patent: **18.06.2025**
(21) Application number: 23165882.4
(22) Date of filing: 31.03.2023
(51) Int. Cl.: G01B 9/02, G01B 9/02055, G01B 9/02091

(54) **LINE-FIELD FOURIER-DOMAIN OPTICAL COHERENCE TOMOGRAPHY IMAGING SYSTEM**
OPTISCHES BILDGEBUNGSSYSTEM FÜR DIE LINIENFELDFOURIERDOMÄNENKOHÄRENZTOMOGRAFIE
SYSTÈME D'IMAGERIE PAR TOMOGRAPHIE PAR COHÉRENCE OPTIQUE DANS LE DOMAINE DE FOURIER À CHAMP LINÉAIRE

(43) Date of publication of application: 02.10.2024
(73) Proprietor: Optos plc, Scotland KY11 8GR (GB)
(72) Inventor: NORMAND, Margaret Catherine, Dunfermline, Scotland, KY11 8GR (GB); PRECIADO, Miguel Angel, Dunfermline, Scotland, KY11 8GR (GB)
(74) Representative: Hoffmann Eitle

(56) References cited:
- US-A1- 2008 231 807
- PANDIYAN VIMAL PRABHU ET AL: "Reflective mirror-based line-scan adaptive optics OCT for imaging retinal structure and function", BIOMEDICAL OPTICS EXPRESS, vol. 12, no. 9, 1 September 2021 (2021-09-01), United States, pages 5865, XP093075653, ISSN: 2156-7085, Retrieved from the Internet <URL:https://www.ncbi.nlm.nih.gov/pmc/articles/PMC8515964/pdf/boe-12-9-5865.pdf> [retrieved on 20230822], DOI: 10.1364/BOE.436337
- LAURIN GINNER ET AL: "Noniterative digital aberration correction for cellular resolution retinal optical coherence tomography in vivo", OPTICA, vol. 4, no. 8, 20 August 2017 (2017-08-20), US, pages 924 - 931, XP055518568, ISSN: 2334-2536, DOI: 10.1364/OPTICA.4.000924
- LIANG SHANSHAN ET AL: "Design and Simulation of an Ultra-Wide Field Optical Coherence Tomography Retinal Imaging System", PHOTONICS, vol. 8, no. 11, 1 November 2021 (2021-11-01), pages 476, XP093075656, DOI: 10.3390/photonics8110476

## Description

### [Field]

Example aspects herein generally relate to the field of optical coherence tomography (OCT) imaging systems and, in particular, to Fourier-domain OCT (FD-OCT) imaging systems.

### [Background]

Optical coherence tomography (OCT) is an imaging technique based on low-coherence interferometry, which is widely used to acquire high-resolution two- and three-dimensional images of optical scattering media, such as biological tissue.

OCT imaging systems can be classified as being time-domain OCT (TD-OCT) or Fourier-domain OCT (FD-OCT) (also referred to as frequency-domain OCT), depending on how depth ranging is achieved. In TD-OCT, an optical path length of a reference arm of the imaging system's interferometer is varied in time during the acquisition of a reflectivity profile of the scattering medium being imaged by the OCT imaging system (referred to herein as the "imaging target"), the reflectivity profile being commonly referred to as a "depth scan" or "axial scan" ("A-scan"). In FD-OCT, a spectral interferogram resulting from an interference between the reference arm and the sample arm of the interferometer at each A-scan location is Fourier transformed to simultaneously acquire all points along the depth of the A-scan, without requiring any variation in the optical path length of the reference arm. FD-OCT can allow much faster imaging than scanning of the sample arm mirror in the interferometer, as all the back reflections from the sample are measured simultaneously. Two common types of FD-OCT are spectral-domain OCT (SD-OCT) and swept-source OCT (SS-OCT). In SD-OCT, a broadband light source delivers many wavelengths to the imaging target, and all wavelengths are measured simultaneously using a spectrometer as the detector. In SS-OCT (also referred to as time-encoded frequency-domain OCT), the light source is swept through a range of wavelengths, and the temporal output of the detector is converted to spectral interference.

OCT imaging systems can also be classified as being point-scan (also known as "point detection" or "scanning point"), line-field or full-field, depending on how the imaging system is configured to acquire OCT data laterally. A point-scan OCT imaging system acquires OCT data by scanning a sample beam, which is focussed to a point on the surface of the imaging target, typically along a single line (which may be straight, or alternatively curved so as to define a circle or a spiral, for example) or along a set of (usually substantially parallel) lines on the surface of the imaging target, and acquiring an axial depth profile (A-scan) for each of a plurality of points along the line(s), one single point at a time, to build up OCT data comprising a one- or two-dimensional array of A-scans representing a two-dimensional or three-dimensional (volumetric) reflectance profile of the sample. A line-scan OCT imaging system acquires OCT data by generating a line of light, for example by shaping a (nominally circular) beam of light into a line of light using a cylindrical lens or a Powell lens, for example, scanning the line of light (i.e. line-field illumination) across the surface of the imaging target, and acquiring in parallel an axial depth profile (A-scan) for each of a plurality of points along the line, for each scan location of the line, to build up OCT data comprising a one- or two-dimensional array of A-scans representing a two-dimensional or three-dimensional (volumetric) reflectance profile of the sample. A full-field FD-OCT imaging system typically uses a swept-source laser or a superluminescent diode (SLD) and a 2D detector array, such as a CCD camera, to acquire OCT data in the form of tomographic images in the enface (transverse) orientation, which are recorded in parallel by the 2D detector array.

In ophthalmic applications, OCT imaging systems may be wide-field (WF) or ultrawide-field (UWF), where an extended region of a retina of an eye (as an example of the imaging target 130) is illuminated. In accordance with accepted definitions of WF and UWF provided by the International Widefield Imaging Study Group (Choudhry N. et al. "Classification and guidelines for widefield imaging: Recommendations from the International Widefield Imaging Study Group", Ophthalmol. Retin. 2019;3:10:843-849), WF retinal images are centred on the fovea and include the retina in all four quadrants posterior to and including the vortex vein ampullae, while UWF retinal images show retinal anatomy anterior to the vortex vein ampullae in all four quadrants. WF imaging systems have an approximately 60- to 100-degree field of view, capturing the mid-periphery of the retina up to the posterior edge of the vortex vein ampulla. UWF imaging systems have an approximately 110- to 220-degree field of view, so as to capture the far periphery of the retina, including the anterior edge of the vortex vein ampulla and beyond.

However, the image quality of such conventional WF and UWF OCT imaging systems can be degraded by the imaging optics typically used in such systems, as well as by movements of the eye (or other imaging target) during image capture. OCT volume scan of the retina made by such systems tend to be too slow to allow capture of a large area of the retina without eye movement. Slow capture also prevents the study of small changes in the retina in response to a stimulus (i.e. functional OCT). Faster acquisition would be important for OCT angiography (OCTA), and relax the requirements on tracking. Accordingly, there remains a need for OCT imaging systems that can capture WF or UWF OCT images that are free of (or at least have less of) the optical aberrations that are typically caused by the WF/UWF imaging optics, and on timescales that are sufficiently short to reduce or eliminate the effects of the image target movements.

Pandiyan VP et al., "Reflective mirror-based line-scan adaptive optics OCT for imaging retinal structure and function", Biomed Opt Express, 2021 Aug 27;12(9):5865-5880 discloses a line-field OCT system comprising a spherical mirror in the scanning system wherein aberrations are corrected using an adaptive optics system. US 2008/231807 A1 discloses a full-field OCT system comprising a parabolic mirror in the scanning system wherein aberrations are corrected using a adaptive optics system.

### [Summary]

There is provided, in accordance with a first example aspect herein, a line-field Fourier-domain optical coherence tomography (OCT) imaging system, comprising: a line-field illumination source arranged to generate a line of light; a scanning system comprising a scanning element and a curved mirror having a first focal point and a conjugate second focal point, the scanning element being located at the first focal point of the curved mirror and arranged to perform a scan of an imaging target via the second focal point of the curved mirror, by scanning at least a segment of the line of light across the imaging target via the curved mirror, the scanning element being further arranged to receive, via the curved mirror, light which has been scattered by the imaging target during the scan and aberrated by the curved mirror to form an aberrated line of light comprising at least one of a defocussing or a distortion. The line-field Fourier-domain OCT imaging system further comprises a photodetector comprising an array of photodetector elements, and an interferometer arranged to receive at least a segment of the aberrated line of light via the scanning element, generate an interference line of light resulting from an interference between a reference light and the at least a segment of the aberrated line of light received via the scanning element, and project the interference line of light onto the array of photodetector elements. The photodetector is arranged to detect the interference line of light projected onto the array of photodetector elements during the scan, and generate a detection signal based on the detected interference line of light. The line-field Fourier-domain OCT imaging system further comprises OCT data processing hardware arranged to: generate complex volumetric OCT data of the imaging target based on the detection signal, wherein the complex volumetric OCT data has an optical aberration therein; and generate corrected complex volumetric OCT data by executing a correction algorithm which uses phase information encoded in the complex volumetric OCT data to correct the complex volumetric OCT data, such that the corrected complex volumetric OCT data has less of the optical aberration than the complex volumetric OCT data. The line-field Fourier-domain OCT imaging system of the first example aspect may be a wide-field (WF) line-field Fourier-domain OCT imaging system or an ultrawide-field (UWF) line-field Fourier-domain OCT imaging system.

In an example embodiment, the line-field Fourier-domain OCT imaging system may be a line-field swept-source OCT imaging system, the line-field illumination source may be a swept line-field illumination source arranged to generate the line of light, and the array of photodetector elements may be a one-dimensional array of photodetector elements, wherein the photodetector elements of the one-dimensional array of photodetector elements are arrayed along a length of a projection of the interference line of light onto the one-dimensional array of photodetector elements. In this example embodiment, the photodetector elements of the one-dimensional array may have a width, in a direction perpendicular to a length of the projection of the interference line of light onto the one-dimensional array of photodetector elements (i.e. perpendicular to a direction along which the projection of the interference line of light extends along the array of photodetector elements, which is wider than a maximum width of the projection of the interference line of light onto the one-dimensional array of photodetector elements.

In an alternative example embodiment, the line-field Fourier-domain OCT imaging system may be a line-field swept-source OCT imaging system, the line-field illumination source may be a swept line-field illumination source, the array of photodetector elements may be a two-dimensional array of photodetector elements, and the photodetector elements of the two-dimensional array of photodetector elements may be arrayed in a first direction along a length of the projection of the interference line of light onto the two-dimensional array of photodetector elements, and in a second direction which is perpendicular to the first direction. In this example embodiment, a width of the two-dimensional array of photodetector elements, in the second direction, may be wider than a maximum width of the projection of the interference line of light onto the two-dimensional array of photodetector elements, and the projection of the interference line of light onto the two-dimensional array of photodetector elements may be spanned in the second direction by a plurality of the photodetector elements.

In another example embodiment, the line-field Fourier-domain OCT imaging system may be a line-field spectral domain OCT imaging system, the line-field illumination source may be a broadband line-field illumination source arranged to generate the line of light, the array of photodetector elements may be a two-dimensional array of photodetector elements, and the photodetector may comprise a two-dimensional spectrometer comprising a diffraction element and the two-dimensional array of photodetector elements, the diffraction element being arranged to disperse a spectral content of the interference line of light across the two-dimensional array of photodetector elements in a first direction, which is perpendicular to a second direction along which a projection of the interference line of light along the array of photodetector elements extends, wherein the photodetector elements of the two-dimensional array are arrayed in the first direction and in the second direction.

In the line-field Fourier-domain OCT imaging system of the first example aspect or any of the example embodiments set out above, the scanning element may be a first scanning element arranged to scan the at least a segment of the line of light in a first direction across a surface of the imaging target such that projections of the at least a segment of the line of light onto the surface of the imaging target during the scan are displaced relative to each other along the first direction, and the scanning system may further comprise a second scanning element, which is arranged to reflect the at least a segment of the line of light from the line-field illumination source toward the first scanning element, and arranged to change, in a second direction that is perpendicular to the first direction, and in a third direction opposite to the second direction, a position at which the first scanning element is to scan the at least a segment of the line of light across the surface of the imaging target. The first scanning element may be arranged to scan the at least a segment of the line of light in the first direction across the surface of the imaging target, or in a direction opposite to the first direction across the surface of the imaging target, both before and after the change.

In the foregoing, the curved mirror may be a (prolate) spheroidal mirror having an axis of circular symmetry, and the scanning element may be arranged to perform the scan of the imaging target by scanning the at least a segment of the line of light across the imaging target via the spheroidal mirror such that the at least a segment of the line of light incident on the spheroidal mirror propagates in a plane which is parallel to the axis of circular symmetry of the spheroidal mirror.

Alternatively, the curved mirror may be a (prolate) spheroidal mirror, and the scanning element may be arranged to perform the scan of the imaging target by scanning the at least a segment of the line of light across the imaging target via the spheroidal mirror such that a portion of the spheroidal mirror, onto which the at least a segment of the line of light is projected, has reflective symmetry about a plane parallel to and passing through the axis of circular symmetry.

The line-field Fourier-domain OCT imaging system of the first example aspect or any of the example embodiments set out above may further comprise a mask having a first region and a second region that surrounds the first region, wherein the first region has a higher transparency than the second region, the mask both being located in the line-field Fourier-domain OCT imaging system and having the first region shaped so as to allow at least some of the interference line of light to propagate to the array of photodetector elements and at least partially prevent light other than the interference line of light from propagating to the array of photodetector elements.

The line-field Fourier-domain OCT imaging system of the first example aspect or any of the example embodiments set out above may further comprise a light source, wherein the line-field Fourier-domain OCT imaging system is arranged to measure a response of a retina of an eye to a light stimulus generated by the light source.

There is provided, in accordance with a second example aspect herein, a full-field swept-source OCT imaging system, comprising: a swept illumination source arranged to generate a beam of light whose wavelength is swept over a range of values during imaging by the full-field swept-source OCT imaging system; a transfer system comprising a curved mirror having a first focal point and a conjugate second focal point, the transfer system further comprising a focusing system which is arranged to focus the beam of light generated by the swept illumination source at the first focal point, the curved mirror being arranged to guide the light beam focused at the first focal point to an imaging target via the second focal point of the curved mirror so as to provide full-field illumination of the imaging target during imaging of the imaging target, the curved mirror being further arranged to receive light which has been scattered by the imaging target during the imaging of the imaging target and aberrated by the curved mirror to form an aberrated beam of light comprising at least one of a defocussing or distortion. The full-field swept-source OCT imaging system further comprises a photodetector comprising a two-dimensional array of photodetector elements, and an interferometer arranged to receive at least a portion of the light which has been received by the curved mirror and to generate an interference light resulting from an interference between a reference light and the at least a portion of the aberrated beam of light, and project the interference light onto the two-dimensional array of photodetector elements. The photodetector is arranged to detect the interference light projected onto the two-dimensional array of photodetector elements during the imaging, and generate a detection signal based on the detected interference light. The full-field swept-source OCT imaging system further comprises OCT data processing hardware arranged to: generate complex volumetric OCT data of the imaging target based on the detection signal, wherein the complex volumetric OCT data has an optical aberration therein; and generate corrected complex volumetric OCT data by executing a correction algorithm which uses phase information encoded in the complex volumetric OCT data to correct the complex volumetric OCT data, such that the corrected complex volumetric OCT data has less of the optical aberration than the complex volumetric OCT data. The curved mirror may be a parabolic mirror, such as an elliptical mirror. The full-field swept-source OCT imaging system may further comprise a light source, and may be arranged to measure a response of a retina of an eye to a light stimulus generated by the light source.

In the first example aspect and/or the second example aspect, or any of the example embodiments thereof set out above, the OCT data processing hardware may be arranged to: generate the complex volumetric OCT data of the imaging target such that the complex volumetric OCT data, when processed to generate an enface projection of the complex volumetric OCT data, provides an enface projection having an optical aberration therein; and generate the corrected complex volumetric OCT data such that the corrected complex volumetric OCT data, when processed to generate an enface projection of the corrected complex volumetric OCT data, provides an enface projection having less of the optical aberration than the enface projection of the complex volumetric OCT data. The OCT data processing hardware may additionally or alternatively be arranged to: generate the complex volumetric OCT data of the imaging target such that at least one B-scan of the complex volumetric OCT data has an optical aberration therein; and generate the corrected complex volumetric OCT data such that at least one corresponding corrected B-scan of the corrected complex volumetric OCT data has less of the optical aberration than the at least one B-scan of the complex volumetric OCT data.

There is provided, in accordance with a third example aspect herein, a computer-implemented method of processing complex volumetric OCT data of an imaging target generated by either the line-scan Fourier-domain OCT imaging system of the first example aspect or the full-field Fourier-domain OCT imaging system of the second example aspect, the method comprising: acquiring the complex volumetric OCT data of the imaging target the complex volumetric OCT data having an aberration therein; and generating corrected complex volumetric OCT data by executing a correction algorithm which uses phase information encoded in the complex volumetric OCT data to correct the complex volumetric OCT data, such that the corrected complex volumetric OCT data has less of the optical aberration than the complex volumetric OCT data.

### [Brief Description of the Drawings]

Example embodiments will now be explained in detail, by way of non-limiting example only, with reference to the accompanying figures described below. Like reference numerals appearing in different ones of the figures can denote identical or functionally similar elements, unless indicated otherwise.
Figure 1 is a schematic illustration of line-field swept-source OCT imaging system according to a first example embodiment herein.
Figure 2 is a schematic illustration of a scanning system forming part of the first example embodiment.
Figure 3A is a schematic illustration of an alternative scanning system, which may form part of the first example embodiment, in place of scanning system of Figure 2.
Figure 3B is a schematic illustration of portion of the scanning system in Figure 3A.
Figure 4A is a schematic illustration of a two-dimensional array of photodetector elements included in the first example embodiment.
Figure 4B is a schematic illustration of a one-dimensional array of photodetector elements, which may be used in place of the two-dimensional array of photodetector elements shown in Figure 4A.
Figure 5 is a schematic illustration of a two-dimensional array of photodetector elements for use in a line-field SD-OCT imaging system according to an alternative embodiment.
Figure 6 is a schematic illustration of a mask that may be used in the first example embodiment and/or the alternative example embodiment.
Figure 7 is a schematic illustration of a programmable signal processing hardware, which may be configured to perform the functions of the OCT data processing hardware described herein.
Figure 8 is a schematic illustration of full-field FD-OCT imaging system according to a second example embodiment herein.
Figure 9 is a flow diagram illustrating a process of generating corrected complex volumetric OCT data according to an example embodiment herein.

### [Detailed Description of Example Embodiments]

In view of background provided above, the present inventors have devised a line-field FD-OCT imaging system and a full-field FD-OCT imaging system which utilise a curved mirror having conjugate focal points to enable light to illuminate an extended region of a retina of an eye, to enable WF or UWF imaging of the eye. Further, by employing an array of photodetector elements to detect (preferably most or all of) the defocused interference light, which has larger projection on the image plane (owing to optical aberrations caused, at least in part, by the curved mirror) than the interference light would have in the absence of the aberrations, phase information in the acquired complex volumetric OCT data can be obtained and used to digitally refocus the complex volumetric OCT data with a numerical correction algorithm so as to mitigate the effects of the optical aberrations.

The use of digital refocusing, combined with the inherently higher imaging speed of line-field and full-field OCT imaging systems, can significantly increase the quality of the OCT images produced. Further, the relatively fast OCT data acquisition speed of OCT systems described herein may enable functional OCT to be performed across a wide of ultrawide field-of-view.

Example embodiments of a line-field FD-OCT imaging system and a full-field FD-OCT imaging system will now be described in detail with reference to accompanying drawings.

### [First Example Embodiment]

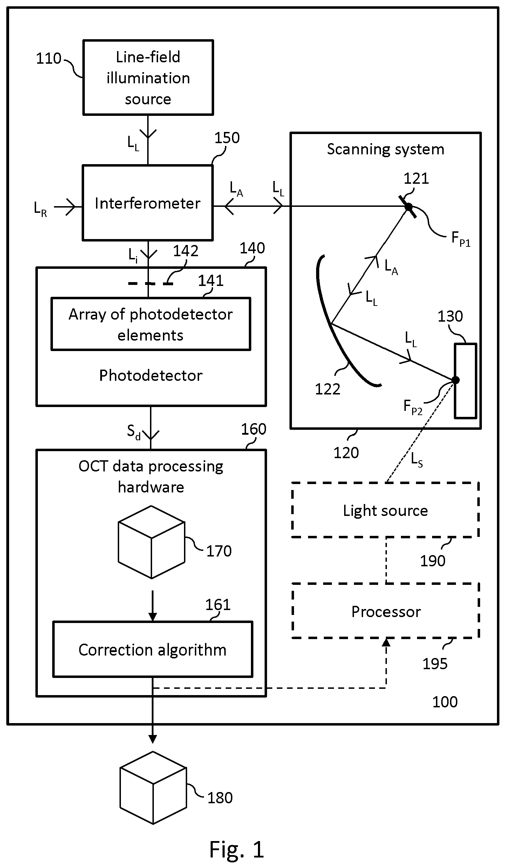

Figure 1 is schematic illustration of a line-field FD-OCT imaging system 100 according to a first example embodiment herein. The line-field FD-OCT imaging system 100 comprises a line-field illumination source 110, a scanning system 120, a photodetector 140, an interferometer 150 and OCT data processing hardware 160. The line-field FD-OCT system 100 may, as in the present example embodiment, be a line-field swept-source OCT (SS-OCT) system. However, the line-field FD-OCT imaging system 100 need not be provided in this form and may, for example, take the alternative form of a spectral-domain OCT (SD-OCT) imaging system. More generally, an example embodiment may be provided as any form of line-field FD-OCT imaging system that can generate complex volumetric OCT data 170, i.e. Fourier transforms of respective spectral interferograms (interference spectra) representing complex A-scan information obtained for each scan line location at which an OCT measurement was made during successive line scans. Such complex volumetric OCT data 170 encodes phase information from acquired OCT measurements that can be used by a correction algorithm 161 as described herein to digitally refocus OCT image data. Further, the line-field FD-OCT imaging system 100 may be a wide-field (WF) or an ultra-wide field (UWF) FD-OCT imaging system, as described below.

The line-field illumination source 110 is arranged to provide line illumination by generating a line of light L_{L}. The line-field illumination source 110 may, as in the present example embodiment, be a swept line-field illumination source, which is arranged to generate a line of light L_{L} having a wavelength that is swept over a range of wavelengths during a scan performed by the line-field FD-OCT imaging system 100. Alternatively, in example embodiments where the line-field FD-OCT imaging system 100 takes the form of a SD-OCT imaging system, the line-field illumination source 110 may be a broadband line-field illumination source, which is arranged to generate a line of light L_{L} simultaneously having a range of wavelengths (i.e. a broad spectral content) during a scan performed by the line-field SD-OCT imaging system.

The line-field illumination source 110 may comprise any known swept or broadband source (as the case may be) and optics arranged to generate the line of light L_{L}. For example, the line-field illumination source 110 may comprise a laser and a one or more cylindrical lenses (e.g. combination of a plano-concave lens and a plano-convex lens that are arranged to focus the beam from the laser in respective directions that are orthogonal to one another to form the line of light L_{L}). However, any other kind of spatial light modulator for beam shaping known to those versed in the art may alternatively be employed. The line-field illumination source 110 may additionally or alternatively employ a diffractive optical element for the beam shaping, or a plurality of light sources arranged in a line to provide the line of light L_{L}.

The scanning system 120 comprises a scanning element 121 and a curved mirror 122 having a first focal point F_{P1} and a conjugate second focal point F_{P2}, wherein light rays passing through the first focal point F_{P1} are reflected by the curved mirror to pass through the second focal point F_{P2}. The scanning element 121 is located at the first focal point F_{P1}, and is arranged to perform a scan of an imaging target 130 via the second focal point F_{P2}, by scanning at least a segment of the line of light L_{L} across the imaging target 130, via the curved mirror 122. The first focal point F_{P1} and the second focal point F_{P2} describe where the long dimension of the line is focussed to a point (the same as the virtual point of a point-scan system). Where the imaging target 130 is a retina of an eye, these focal points may be conjugated with the pupil such that the line can enter the eye. The focal points for the perpendicular line dimension may be in different planes, conjugated with the retina. The scanning element 121 may, as in the present example embodiment, comprise a mirror which is arranged to rotate about an axis of rotation passing through the first focal point F_{P1} to scan the line of light L_{L} (or a segment thereof), which is incident on the mirror, across the imaging target 130, via the curved mirror 122. During the scan, the scanning element 121 is further arranged to receive, via the curved mirror 122, light which has been scattered by the imaging target 130 and aberrated by the curved mirror 122 to form an aberrated line of light L_{A} comprising a defocussing and/or a distortion of the light which has been scattered by the imaging target 130 during the scan. The scanning element 121 is preferably large enough to reflect the entire line of light incident thereon. The scanning system 120 and the OCT data processing hardware 160 are thus arranged to acquire B-scans at respective scan lines (or scanning locations) that are distributed across a surface of the imaging target 130, to form complex volumetric OCT data (i.e. an OCT C-scan) by sequentially illuminating the scan lines with the at least a segment of the line of light L_{L}, one scan line at a time, and receiving at least some of the light scattered by the imaging target 130 at each scan line via the curved mirror 122. Although the scanning system 120 may, as in the present example embodiment, perform the scan by sequentially providing line-field illumination along a set of parallel, adjacent scan lines of the same length, the scan pattern used is not so limited.

In ophthalmic applications, the curved mirror 122 may allow the line-field FD-OCT imaging system 100 to function as a WF OCT imaging system, by allowing the line of light L_{L} to illuminate a region of the fundus corresponding to an approximately 60- to 100-degree field of view, so as to capture the mid-periphery of the retina up to the posterior edge of the vortex vein ampulla. Alternatively, the curved mirror 122 may allow the line-field FD-OCT imaging system 100 to function as a UWF OCT imaging system, by allowing the line of light L_{L} to illuminate a region of the fundus corresponding to an approximately 110- to 220-degree field of view, so as to capture the far periphery of the retina, including the anterior edge of the vortex vein ampulla and beyond.

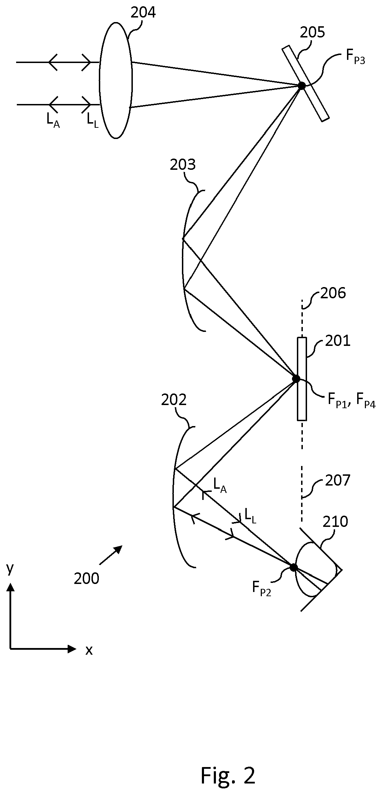

The scanning system 120 may, as in the present example embodiment, include an arrangement of optical components as illustrated schematically in Figure 2. The scanning system 200 shown in Figure 2 comprises a first scanning element 201 and a first curved mirror 202 which are examples of the scanning element 121 and the curved mirror 122 of Figure 1, respectively. The scanning system 200 may, as in the present example, further comprise a second curved mirror 203 (also referred to herein as a "slit mirror"), a lens 204 (e.g. a cylindrical lens) and a second scanning element 205, although these elements are optional, as described below. The second scanning element 205 may, as in the present example embodiment, be in a plane that is conjugate with the plane in which the pupil of the eye 210 is located. During operation of the scanning system 200, at least a segment of the line of light L_{L} enters the scanning system 200 from the interferometer 150 and is focussed by the lens 204 onto the second scanning element 205. However, it is noted that the lens 204 is optional and may be omitted. In this case, the line of light L_{L} from the line-field illumination source 110 (or a segment thereof) may be focussed by an optical element forming a part of the interferometer 150, before being received by the scanning system 200 so as to be in focus at the second scanning element 205. The line of light L_{L} (or segment thereof) received by the second scanning element 205 is then reflected, in sequence, by the second scanning element 205, the second curved mirror 203, the first scanning element 201 and the second curved mirror 202, before being incident on the imaging target 130. As shown in Figure 2, the imaging target 130 takes the form of a region of a retina of an eye 210 in the present example embodiment, although this form of imaging target 130 is given by way of an example only. The return light, which has been scattered by the illuminated region of the retina of the eye 210, follows the same optical path through the scanning system 200 as the line of light L_{L} that is incident on the eye 210 but in reverse order, and exits the scanning system 200 as the aberrated line of light L_{A}, comprising the defocussing and/or a distortion caused by the first curved mirror 202. At least a segment of the aberrated line of light L_{A} is received by the interferometer 150 shown in Figure 1.

The first scanning element 201 is arranged to scan the line of light L_{L} (or the received segment thereof) in a first direction across a surface of the imaging target 130 such that projections of the line of light L_{L} (or the segment thereof) onto the surface of the imaging target 130 during the scan are displaced relative to each other along the first direction. This scan may be performed by the first scan element 201 rotating around the first axis 206 illustrated in Figure 2, to scan the line of light L_{L} (or the segment thereof) in the first direction across the imaging target 130. The first direction may, as in the present example embodiment, be perpendicular to the projections of the line of light L_{L} (or the segment thereof) on the surface of the imaging target 130, i.e. perpendicular to the direction of elongation of the projections of the line of light L_{L}. The first direction is not so limited, however, and may have a component in the direction along the projections of the line of light L_{L} (or the segment thereof) on the surface of the imaging target 130.

The second scanning element 205 is arranged to reflect the received line of light L_{L} (or received segment thereof) toward the first scanning element 201, and to change, in a second direction that is perpendicular to the first direction, and in a third direction opposite to the second direction, a position at which the first scanning element 201 is to scan the line of light L_{L} (or segment thereof) across the surface of the imaging target 130. To do this, the second scan element 205 rotates around a second axis (not shown) to change the position at which the first scanning element 201 is to scan the line of light L_{L} (or segment thereof) across the surface of the imaging target 130 in either of the second direction or the third direction. Thus, by rotating the second scanning element 205, it is possible to translate the line of light L_{L}, in the second direction or the third direction, to a starting scan line position on the imaging target 130. The location along an axis extending along the second and third direction on the surface of the imaging target 130, at which the scan by the first scanning element 210 is to be performed, can thus be set by the orientation of the second scanning element 205. The rotations of the first scanning element 201 and the second scanning element 205 may be controlled by a scanning system controller (not shown).

The first scanning element 201 may, as in the present example embodiment, be arranged to scan the line of light L_{L} (or the receive segment thereof) in the first direction across the surface of the imaging target 130, or in a direction opposite to the first direction, before and after the change described above. Thus, the line-field SS-OCT imaging system 100 of the present example embodiment may scan a first region of the retina of the eye 210 (or more generally, a first region of the imaging target 130), before the second scanning element 205 changes the position at which the first scanning element 201 is to continue scanning the line of light L_{L} (or segment thereof) across the surface of the imaging target 130 in a subsequent scan performed by the first scanning element 201, and the first scanning element 210 then performs a scan over a second region of the imaging target 130 which may partially overlap the first region or be adjacent to the first region. In this way, the line-field FD-OCT imaging system 100 of the present example embodiment may scan two or more different regions of the retina of the eye 210 or other imaging target 130, and combine the acquired OCT data to create a mosaic of OCT data covering a greater portion of the imaging target 130 than a portion covered by any one of the scans individually, thus effectively increasing the field-of-view of the image acquired by the line-field FD-OCT imaging system 100.

The first scanning element 201 and the second scanning element 202 may, as in the present example embodiment, each be a galvanometer optical scanner (a "H-galvo" and a "V-galvo", respectively), although another type of scanning element could alternatively be used, such as a MEMS scanning mirror or a resonant scanning mirror, for example.

The first curved mirror 202 and the second curved mirror 203 may, as in the present example embodiment, be a spheroidal mirror and an ellipsoidal mirror, respectively, each having a first focal point and a conjugate second focal point. The first scanning element 201 is located at the first focal point F_{P1} of the first curved mirror 202 and the imaging target 130 is located in a vicinity of the second focal point F_{P2} of the first curved mirror 202. Where the imaging target 130 is a portion of a retina of an eye 210, as in the present example embodiment, the pupil of the eye 210 is located at the second focal point F_{P2} of the first curved mirror 202 such that the at least a segment of the line of light L_{L} is scanned across a region of the retina of the eye 210 during the scan. The second scanning element 205 is located at the first focal point F_{P3} of the second curved mirror 203, and the first scanning element 201 is located at the second focal point F_{P2} of the second curved mirror 203. However, the second curved mirror 203 (the ellipsoidal mirror in the present example embodiment) may be any reflective component having an aspherical reflective surface, such as a shape of a conical section like a parabola or hyperboloid, or may, more generally, have a shape described by one or more polynomial functions of two variables.

The first curved mirror 202, which has a concave reflective surface in the shape of a prolate spheroid, has an axis of circular symmetry 207. The first scanning element 201 is arranged to perform the scan of the retina of the eye 210 by scanning the line of light L_{L} (or the received segment thereof) across the imaging target 130 via the first curved mirror 202 such that the line of light L_{L} or segment thereof incident on the first curved mirror 202 propagates in a plane which is parallel to the axis of circular symmetry 207 of the first curved mirror 202. In other words, a portion of the first curved mirror 202, onto which the line of light L_{L} (or segment) is projected, has the shape of the ellipse which, when rotated about the axis of circular symmetry 207, forms the spheroid that defines the shape of the first curved mirror 202. To achieve this, the first scanning element 201 rotates about the first axis 206 which is parallel to the axis of circular symmetry 207, as shown in Figure 2.

The light received via the first curved mirror 202, which has been scattered by the imaging target 130 during the scan, is aberrated by the first curved mirror 202 to form the aberrated line of light L_{A} comprising the defocussing and/or distortion. The aberration by the first curved mirror 202 may be a defocussing that is caused by the elliptical curvature of the first curved mirror 202 along the axis of circular symmetry 202, which is in a vertical direction in the present example embodiment. Thus, when a vertical (or diagonal) line field is used for illumination, the full length of the line will not all be in focus at the photodetector 140. Accordingly, although the elliptical curvature of the first curved mirror 202 allows the FD-OCT imaging system 100 to have a wide or ultrawide field-of-view, it also aberrates the return line of light, first upon the line of light L_{L} being reflected off the first curved mirror 202, and secondly by the light which has been scattered by the imaging target 130 being reflected by the first curved mirror 202 before arriving at the first focal point of the first curved mirror 202. Additionally, where the second curved mirror 203 is provided, this may (depending on its shape) cause a similar defocussing of light which it reflects. Thus, the aberrated line of light L_{A} that exits the scanning system 200 may comprise the defocussing from the first curved mirror 202 and a defocussing from the second curved mirror 203. Either or both the first curved mirror 202 and second curved mirror 203 may also cause distortions within the aberrated line of light L_{A} that exits the scanning system 200.

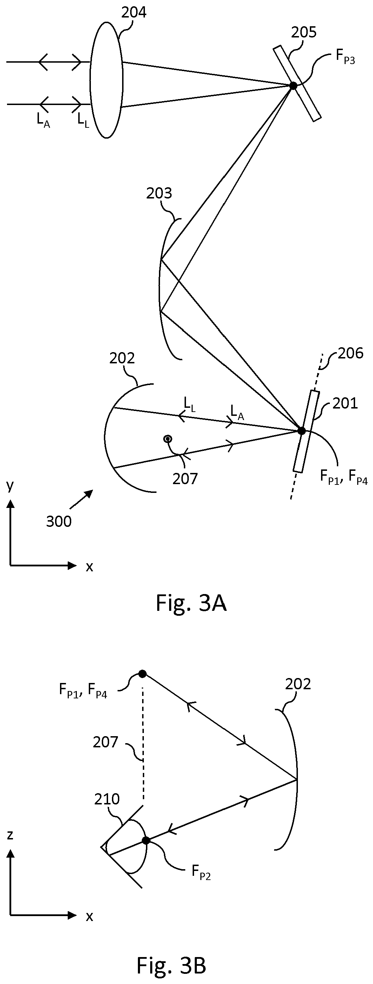

As an alternative to the example implementation described above with reference to Figure 2, the scanning system 120 of Figure 1 may take the alternative form which will now be described with reference to Figures 3A and 3B, which show views in the x-y plane and the x-z plane, respectively, of a scanning system 300 according to an alternative implementation. While the scanning system 120 is arranged to perform vertical scans, and the alternative form of the scanning system 120 described below is arranged to perform horizontal scans, it is noted that any arbitrary scan orientation is possible.

The scanning system 300 differs from scanning system 200 by the orientation of the rotational symmetry axis 207 of the first curved mirror 202 relative to the other optical components of the scanning system, which allows the scanning system 300 to perform "horizontal" scan line scans of the eye (in contrast to the "vertical" scan line scans that are performed by scanning system 200). The form and arrangement of these other optical components, which are identified with the same reference numerals in Figures 2, 3A and 3B, are the same in scanning systems 200 and 300. Whereas the axis 207 is parallel to the y-axis in scanning system 200, as shown in Figure 2, the axis 207 is parallel to the z-axis in scanning system 300, as shown in Figures 3A and 3B. In scanning system 300, the scanning element 201 is arranged to perform the scan of the retina of the eye 210 by scanning the line of light L_{L} (or a segment thereof) across the retina via the first curved mirror 202 such that a portion of the first curved mirror 202, onto which the line of light L_{L} (or segment) is projected, has reflective symmetry about a plane which is parallel to and contains the axis of circular symmetry 207. In Figure 3A, this plane is parallel to the x-z plane and contains the axis 207. Put another way, the scanning element 201 may scan the line of light L_{L} (or a segment thereof) across the retina via the first curved mirror 202 such that the line of light L_{L} (or the segment thereof) incident on the first curved mirror 202 propagates in a plane which contains a semi-axis of the first curved mirror 202 (the semi-axis being a line that defines a radius of the first curved mirror 202 at a point along the axis of circular symmetry 207 of the first curved mirror 202). To achieve this, the first scanning element 201 may rotate about the first axis 206 which is parallel to the semi-axis. As shown in Figure 3B, which shows a portion of the scanning system 300 in the x-z plane, the planes of propagation of the at least a segment of the line of light L_{L} incident on and reflected by the mirror 202 are orthogonal to the x-z plane, within which the axis of circular symmetry of the mirror 202 lies.

By the above arrangement of the first curved mirror 202 in the scanning system 300, the line of light L_{L} and the light scattered by the imaging target 130 are reflected by the first curved mirror 202 such that an illuminated portion of the first curved mirror 202, which makes these reflections, has a substantially less aspherical curvature than the reflecting portion of the first curved mirror 202 in scanning system 200. Accordingly, the defocussing caused by the elliptical curvature of the first curved mirror 202 can be smaller in scanning system 300 than in scanning system 200. However, as the first scanning element 201 rotates about the axis 206, it scans the line of light L_{L} at differing elevations on the first curved mirror 202, where the curvature of the mirror 202 varies, so that the scanning system 300 may need to be refocussed as the elevation changes, to account for the changing curvature of the mirror 202 at each subsequent scan line position. Such refocussing may be difficult to achieve on the required short timescales, and thus a degree of defocussing induced by the elliptical curvature may prevail in scanning system 300. However, this defocussing may be corrected digitally in the same manner as for scanning system 200, as described below.

Although the first curved mirror 202 is a spheroidal mirror in the scanning systems described above with reference to Figures 2, 3A and 3B, it may instead be any reflective component having an aspherical shape, such as a shape of a conical section like a parabola or hyperboloid, or may, more generally, have a shape described by one or more polynomial functions of two variables. Further, the inclusion of the second scanning element 205 and the second curved mirror 203 are optional, and these optical components may be removed such that the first scanning element 201 is placed at the focal point of the cylindrical lens 204.

Where the FD-OCT imaging system 100, which comprises the scanning system 200 of Figure 2 (or the alternative scanning system 300 of Figures 3A and 3B) is an ophthalmic FD-OCT imaging system, the FD-OCT imaging system 100 may be arranged to acquire OCT data from an imaging target 130 in the form of the region of the retina of the eye 210, although any other part of the eye 210 that can be imaged by OCT, such as a portion of the anterior segment of the eye, may alternatively or additionally be imaged by the FD-OCT imaging system 100. The imaging target 130 is not, however, limited to a portion of an eye, and may alternatively be any tissue (e.g. skin), biological sample or, more generally, any scattering medium whose sub-subsurface structure is to be imaged by OCT.

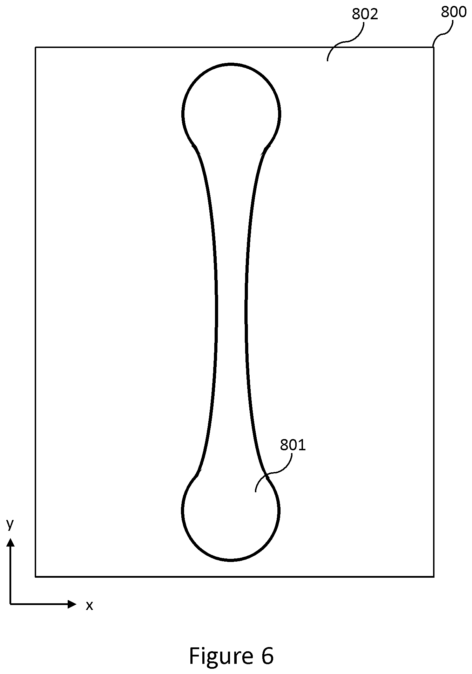

Referring again to Figure 1, the photodetector 140 comprises an array of photodetector elements 141, whose light-receiving surfaces are in a plane conjugate with the retina of the eye 210. The array 141 may, as in the present example embodiment, comprise a plurality of photodetector elements arranged in a two-dimensional grid of orthogonal rows and columns. However, the array 141 may, more generally, be a one-dimensional or any two-dimensional array of photodetector elements. The photodetector elements may be photodiodes, phototransistors, or capacitors of a charge-coupled device, for example. The FD-OCT imaging system 100 may, as in the present example embodiment, further comprise a mask 142, an example of which is described in more detail below, with reference to Figure 6. The mask 142 may be provided as part of the photodetector 140 (as shown in Figure 1) or as a component that is external to the photodetector 140.

The interferometer 150 is a free-space interferometer, which is arranged to split the line of light L_{L} from the line-field illumination source 110 to propagate along a sample arm and a reference arm of the interferometer 150. The interferometer 150 is further arranged to receive at least a segment of the aberrated line of light L_{A} via the scanning element 121, generate an interference line of light L_{I} resulting from an interference between a reference line of light L_{R} from the reference arm and the at least a segment of the aberrated line of light L_{A} in the sample arm, which has been received via the scanning element 121, and project (preferably the whole of) the interference line of light L_{I} onto the array of photodetector elements 141. In other words, the reference light L_{R} and the least a segment of the aberrated line of light L_{A} received by the interferometer 150 from the scanning system 120 during the scan are guided to coincide and interfere with one another, and the resulting interference line of light L_{I} is directed to and received by the array of photodetector elements 141. The interferometer 150 may, as in the present example embodiment, be a Michelson interferometer using a beam-splitter to split the line of light L_{L} to propagate along the sample arm and reference arm of the interferometer, and to interfere the reference light L_{R} and the at least a segment of the aberrated line of light L_{A}. However, any free-space interferometer suitable for line-field OCT may be used, such as a Mach-Zehnder interferometer, for example. The optical path from the imaging target 130 to the photodetector 140, including the interferometer 150, may, as in the present example embodiment, be entirely free space.

Thus, the photodetector 140 is arranged to detect the interference line of light L_{I} projected onto the array of photodetector elements 141 during the scan, and generate a detection signal S_{d} based on the detected interference line of light L_{I}. The photodetector 140 generates the detection signal S_{d} by performing a photoelectric conversion of the interference line of light Lᵢ that is incident on the photodetector elements. The specific form of the photodetector 140 depends on the form in which the line-field FD-OCT is implemented.

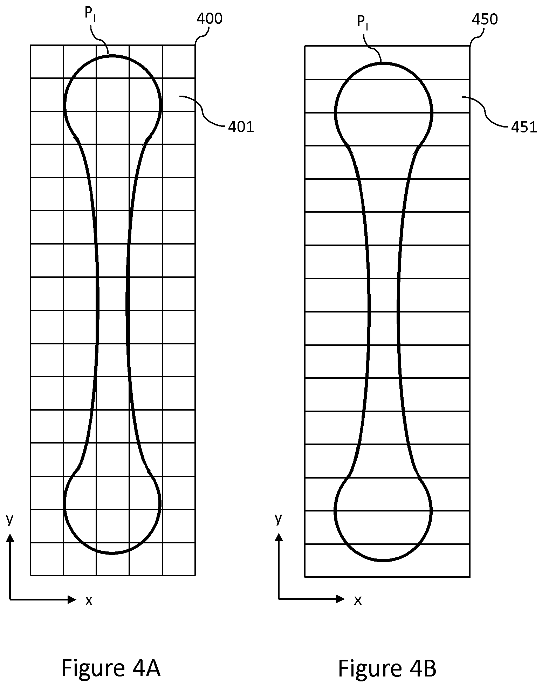

Where the line-field FD-OCT imaging system 100 is a line-field SS-OCT imaging system, as in the present example embodiment, the array of photodetector elements 141 may, as in the present example embodiment, be a two-dimensional array of photodetector elements 400, as illustrated schematically in Figure 4A. The photodetector elements 401 of the two-dimensional array of photodetector elements 400 are arrayed in a first direction (the y-axis direction) along a length of the projection P_{I} of the interference line of light L_{I} onto the two-dimensional array of photodetector elements 400, and in a second direction (the x-axis direction), which is perpendicular to the first direction. The projection P_{I} has a shape caused by the defocusing and/or distortion in the aberrated line of light L_{A}.

By way of an example, the projection P_{I} shown in Figure 4A has a shape of a line segment having a line width which gradually increases from the centre of the line segment towards the ends of the line segment due to the light which has been scattered by the imaging target 130 during the scan being aberrated, at least in part, by the first curved mirror 202 in the scanning system 200, as described above.

The width of the two-dimensional array of photodetector elements 400 in the second direction may, as in the present example embodiment, be greater than a maximum width of the projection P_{I}, and the projection P_{I} may be spanned in the second direction by a plurality of the photodetector elements 401, as illustrated in Figure 4A. The maximum width of the projection P_{I} may, as in the present example embodiment, be the displacement in the second direction between the furthest point in the second direction, at which light from the interference line of light Lᵢ is detectable by the photodetector elements (i.e. where the light intensity is above a minimum light intensity detectable by the photodetector elements) and the furthest point in a third direction, opposite to the second direction, at which light from the interference line of light Lᵢ is detectable by the photodetector elements (i.e. where the light intensity is above the minimum light intensity detectable by the photodetector elements). This is illustrated schematically in Figure 4A by the boundary of the projection P_{I} of the interference line of light L_{I}, along which the intensity of the interference line of light L_{I} equals the minimum light intensity detectable by the photodetector elements. As shown in Figure 4A, the photodetector elements extend beyond the boundaries of the projection P_{I} of the interference line of light Lᵢ in the first and second directions so that all detectable interference light can be detected by the photodetector array 400. In other words, the projection P_{I} of the interference line of light Lᵢ onto a plane containing the surface of the array of photodetector elements 401 is surrounded by a periphery of the surface of the array of photodetector elements 401. It is noted that the maximum width of the projection P_{I} may be defined in other ways, for example as the largest width out of the widths, in the second direction, of the interference line of light Lᵢ between locations along the first direction (or x-axis) at which the intensity of the interference line of light Lᵢ has fallen to 0.1 %, 1 %, 5% or 10 % of the peak intensity value in the second direction at the respective location in the first direction (or the peak intensity value of the whole of the projected interference line of light L_{I}).

While the two-dimensional array of photodetector elements 400 is shown in Figure 4A as a square grid of photodetector elements (with equal spacing between photodetector elements along the two directions in which they are arrayed), the form of the array is not so limited, and may take any alternative form. The photodetector elements may, for example, be arranged in a rectangular grid or triangular grid instead.

As an alternative to the two-dimensional array of photodetector elements 400 illustrated in Figure 4A, the photodetector array 141 of the photodetector 140 may be provided in the form of a one-dimensional array of photodetector elements 450, as illustrated schematically in Figure 4B. The photodetector elements 451 of the one-dimensional array of photodetector elements 450 are arrayed along the length of the projection P_{I} of the interference line of light L_{I} onto the one-dimensional array of photodetector elements 450 (i.e. in the first direction describe above, along the y-axis).

The photodetector elements of the one-dimensional array 450 may have a width, in a direction perpendicular to a length of the projection P_{I} of the interference line of light L_{I} onto the one-dimensional array of photodetector elements 450 (i.e. in the second direction described above, which is along the x-axis shown in Figure 4B), which is greater than a maximum width of the projection P_{I} in the second direction. The maximum width may be defined in manner described above with respect to Figure 4A. The one-dimensional array of photodetector elements 450 may be centred in the first direction at the halfway point of the maximum width in the first direction, as shown in Figure 4B.

The photodetector 140, when provided in the form of photodetector 400 or photodetector 450 described above, maximise the detection of the interference light that exits the interferometer 150. Accordingly, a photodetector of these kinds may effectively detect the interference between the reference light L_{R} and the aberrated line of light L_{A}, which includes the defocussing and/or distortion. The detection signal S_{d} generated by the photodetector 140 thus contains information about the defocussing and/or distortion (more specifically, information on how the measured intensity and phase are spatially distributed), which can be used by the correction algorithm 161 executed by the OCT data processing hardware 160 to digitally correct the defocussing and/or distortion in the acquired OCT data 170, as described below.

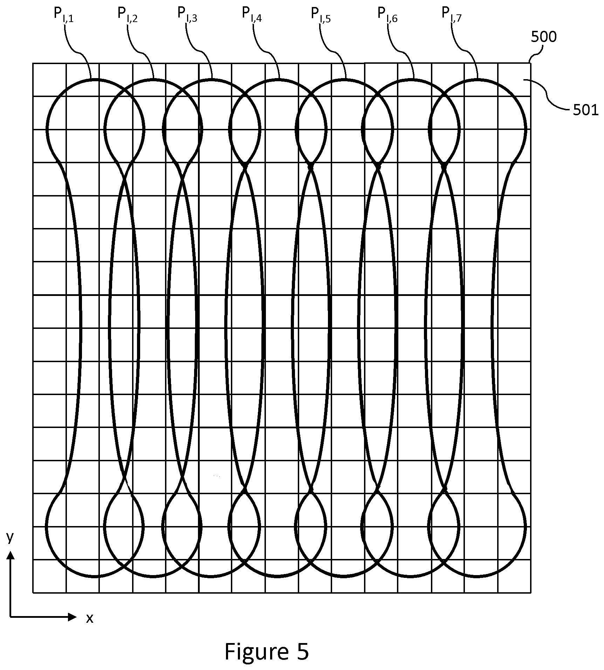

Where the line-field FD-OCT imaging system 100 is a line-field SD-OCT imaging system, as described above, the photodetector 140 may comprise a two-dimensional spectrometer comprising a diffraction element and a two-dimensional array of photodetector elements, as the array of photodetector elements 141. Figure 5 is a schematic illustration of a two-dimensional array of photodetector elements 500 forming part of the two-dimensional spectrometer. The diffraction element (not shown) is arranged to disperse a spectral content of the interference line of light L_{I} across the two-dimensional array of photodetector elements 500 in the second direction (along the x-axis), which is perpendicular to the first direction (along the y-axis) along which the projections of the interference line of light L_{I} extend, as shown in Figure 5 (for illustrative purposes, only a selection of seven projections, P_{I,1} to P_{I,7}, from a continuum of projections created by the diffraction element, are shown in Figure 5). The photodetector elements 501 of the two-dimensional array of photodetector elements 500 are arrayed in the first direction and in the second direction. The spectral content of the interference line of light L_{I} that is projected by the diffraction grating onto the two-dimensional array of photodetector elements 500 during the scan comprises overlapping projections of interference light of varying wavelengths, whose spatial distributions are defined, at least in part, by the defocusing and/or distortion within the aberrated line of light L_{A}. By way of example, in Figure 5, the projections P_{I,1} to P_{I,7} corresponding to a selection of wavelengths of the interference line of light Lᵢ are shown to have a shape of a line segment having a line width which gradually increases from the centre of the line segment towards the ends of the line segment, due to the light, which has been scattered by the imaging target 130 during the scan, being aberrated, at least in part, by the curved mirror 202 in the scanning system 200, as described above.

While the two-dimensional array of photodetector elements 500 is shown in Figure 5 as a square grid of photodetector elements, the form of the array is not so limited, and may take any alternative form. The photodetector elements may, for example, be arranged in a rectangular grid or triangular grid instead.

In Figures 4A, 4B and 5, the numbers and sizes of the photodetector elements have been chosen to facilitate explanation of the principles herein and do not necessarily reflect the numbers and sizes required in practical implementations of the photodetector 140. In practical implementations of the photodetector 140, the photodetector elements will typically be much smaller than the linewidth of the projection P_{I} of the interference line of light L_{I}, and many more photodetector elements would usually span the projection P_{I} than illustrated in Figures 4A, 4B and 5.

The mask 142 (where provided) has a first region and a second region that surrounds the first region, wherein the first region has a higher transparency than the second region. The mask 142 is both located in the line-field Fourier-domain OCT imaging system 100 and has the first region shaped so as to allow at least some of the interference line of light L_{I} to propagate to the array of photodetector elements 141 and at least partially prevent light other than the interference line of light L_{I} from propagating to the array of photodetector elements 141. The location of the mask 142 in line-field Fourier-domain OCT imaging system 100 may, as in the present example embodiment, be in a plane conjugate to the imaging target 130 (which may be a plane in front of the photodetector 140) and the shape of the first region may be chosen such that, when placed within the conjugate plane, at least some of the interference line of light L_{I} is allowed by the mask 142 to propagate to the array of photodetector elements 141, and light other than the interference line of light L_{I} is at least partially prevented by the mask 142 from propagating to the array of photodetector elements 141. Thus, the first region of the mask 142 of the higher transparency allows the at least some of the interference line of light L_{I} to propagate therethrough to the array of photodetector elements 141, and the second region of the mask 142, which has the lower transparency (and which may be opaque), at least partially prevents unwanted light, such as unwanted reflections or artifacts, from propagating to the array of photodetector elements 141. The mask 142 therefore filters the unwanted signals from the interference line of light L_{I}, which may improve the quality (including the signal-to-noise ratio) of the detection signal S_{d}. The shape of the first region may be optimized to maximise the signal-to-noise ratio while minimizing the artifacts due to the light from areas different to the imaging target 130.

The mask 142 of Figure 1 may, for example, be provided in the form of a mask 800 as illustrated in Figure 6, which has a first region 801 and a second region 802. The first region 801 of the mask 800 is a hole within the second region 802 of the mask 800, which is made of an opaque material. However, the form of the mask 800 is not so limited and may alternatively be provided in the form of a graduated neutral density (ND) filter or a spatial light modulator, with is arranged to form the first region 801 with a higher transparency than the second region 802. The mask 800 has a shape which is (approximately) the same as a shape of an outline of a projection of the interference line of light L_{I} onto the array of photodetector elements 141 (the outline of the projection may be defined as a boundary along which the intensity of the interference line of light L_{I} equals the minimum light intensity detectable by the photodetector elements). For example, where the line-field Fourier-domain OCT imaging system 100 is a line-field SS OCT imaging system, the shape of the first region 801 may have the shape of the outline of projection P_{I} in Figure 4A or 4B, and the mask 800 may be placed (in a plane conjugate with the imaging target 130) between the interferometer 150 and the respective array of photodetector elements 400, 450 in Figure 4A or 4B. Where the line-field Fourier-domain OCT imaging system 100 is a line-field SD OCT imaging system, the shape of the first region 801 may have the shape of an outline of the projection P_{I,1} (or any of one of the projections P_{I,1} to P_{I,7}) in Figure 5, and the mask 800 may be placed (in a plane conjugate with the imaging target 130) between the interferometer 150 and the diffraction element of the two-dimensional spectrometer. In either case, the mask 800 may be aligned with the interference line of light L_{I} such that the shape of the first region 801 is overlaid on and aligned with a cross section of the interference line of light L_{I} (which is the shape which determines the shape of the first region 801).

Referring again to Figure 1, the OCT data processing hardware 160 is arranged to generate complex volumetric OCT data 170 of the imaging target 130, based on the detection signal S_{d}, using well-known data processing techniques. The complex volumetric OCT data 170 generated by the OCT data processing hardware 160 based on the detection signal S_{d} may, as in the present example embodiment, comprise a sequence of B-scans, where each B-scan is a two-dimensional array of OCT data elements representing OCT measurement results acquired in a plane through the imaging target 130 that extends in a propagation direction of the line of light L_{L} and a direction orthogonal to the propagation direction. The complex volumetric OCT data 170 has an optical aberration therein, such as a defocusing (blurring) and/or a distortion. The optical aberration may originate in some of the optics within the FD-OCT imaging system 100, for example in one of more curved mirrors that may be provided in the scanning system 120, as described above. That is, at least some of the optical aberration may be caused by the at least one of the defocusing or the distortion in the aberrated line of light L_{A}. Additional optical aberration may be caused by optical imperfections in the imaging target (e.g. the eye in ophthalmic applications of the line-field FD-OCT imaging system 100). Similarly, the complex volumetric OCT data 170, when (or if) processed to generate an enface projection of the complex volumetric OCT data 170 (using any well-known projection technique, such as summed-voxel projection (SVP), or restricted SVP (RSVP), which restricts the SVP to a selected slab of the imaged sample), may provide an enface projection having the optical aberration therein. In other words, the complex volumetric OCT data 170 includes a component (e.g. phase errors, as discussed below) which, when (or if) the complex volumetric OCT data 170 (or only a subset thereof) is processed to generate an enface projection of the complex volumetric OCT data 170 (using any well-known projection technique, as noted above), provides the optical aberration in the enface projection image.

The OCT data processing hardware 160 is further arranged to generate corrected complex volumetric OCT data 180 by executing a correction algorithm 161, which processes phase information encoded in the complex volumetric OCT data 170 to correct the complex volumetric OCT data 170, such that the corrected complex volumetric OCT data 180 (or at least one corrected B-scan of the corrected complex volumetric OCT data 180 which corresponds to the at least one B-scan of the complex volumetric OCT data 170 which has the optical aberration therein) has less of the optical aberration (i.e. a smaller degree (magnitude) of defocusing and/or distortion) than the complex volumetric OCT data 170 (or the at least one B-scan of the complex volumetric OCT data 170 which has the optical aberration therein). Put another way, the OCT data processing hardware 160 is further arranged to generate corrected complex volumetric OCT data 180 by executing a correction algorithm 161, which processes phase information encoded in the complex volumetric OCT data 170 to remove or reduce at least some of the aforementioned component (i.e. the source of the optical aberration present in the complex volumetric OCT data 170 or the at least one B-scan thereof) from the complex volumetric OCT data 170 (or the least one B-scan). As described above, the corrected complex volumetric OCT data 180 generated by the OCT data processing hardware 160 may, as in the present example embodiment, comprise a corresponding sequence of corrected B-scans (to the uncorrected sequence of B-scans). In FD-OCT, complex data encoding the phase information can be obtained from a discrete Fourier transform (DFT) of the optical spectrum of the interference as measured by the line-field FD-OCT imaging system 100.

Similarly, the OCT data processing hardware 160 may be arranged to generate the complex volumetric OCT data 170 of the imaging target 130 such that if the complex volumetric OCT data 160 were to be processed to generate an enface projection of the complex volumetric OCT data 170, the enface projection would have an optical aberration therein, and further arranged to generate the corrected complex volumetric OCT 180 data such that if the corrected complex volumetric OCT data 180, were to be processed to generate an enface projection of the corrected complex volumetric OCT data 180, the resulting enface projection would have less of the optical aberration than the enface projection of the complex volumetric OCT data 170 (as described above).

The OCT data processing hardware 160 thus takes the complex volumetric OCT data 170 as an input to the correction algorithm 161, which is run to generate and output corrected complex volumetric OCT data 180, such that the corrected complex volumetric OCT data 180 (or the one or more corrected B-scans or the corrected enface projection of the corrected complex volumetric OCT data 180) has less of the optical aberration than the input complex volumetric OCT data 170 (or the one or more B-scans or the enface projection thereof). The corrected complex volumetric OCT data 180 (or the one or more corrected B-scans or the corrected enface projection) thus has at least some of the above-mentioned component (source) from the complex volumetric OCT data 170 removed or reduced. As a result, enface projections (or one or more B-scans) of the corrected OCT data 180 may show a decrease in the optical aberration, and so an improved lateral resolution relative to enface projections (or the corresponding one or more B-scans) of the complex volumetric OCT data which serves as an input to the correction algorithm 161. The degree of (de)focussing in an enface projection image or a B-scan, may be quantified using any one of a number of different ways known to those versed in the art. By way of example, the degree of defocusing may be quantified using a gradient-based, Laplacian-based, wavelet-based, statistics-based or discrete cosine transform-based focus measure operators. Various examples of such focus measure operators are provided in the article titled "Analysis of focus measure operators from shape-from-focus" by S. Pertuz et al., published in Pattern Recognition 46 (2013), pages 1415-1432.

The correction algorithm 161 may be any numerical refocusing and/or aberration correction algorithm known in the art, which can improve the lateral resolution (and therefore the effective axial resolution) and/or the contrast in the corrected volumetric OCT data 180 (or an enface projection image or B-scan(s) thereof) by use of complex field information in the volumetric OCT data 170. Generally, the correction algorithm 161 applies a phase filter to the complex volumetric OCT data 170 in the Fourier domain, before taking the inverse Fourier transform of the result. The phase filter is selected to reduce or remove phase errors which would produce the defocusing/distortion in an enface projection of the complex volumetric OCT data 170. An appropriate phase filter can be derived from a mathematical model of the line-field FD-OCT imaging system 100.

By way of an example, the correction algorithm 161 may take the form of the fully automated aberration correction algorithm as described in the article titled "Computational high-resolution optical imaging of the living human retina" by N. D. Shemonski et al., Nature Photonics Vol. 9 (2015): pp. 440-443.

In the described aberration correction algorithm, a Fourier transform of the complex volumetric OCT data is multiplied by a phase filter, before an inverse Fourier transform is applied. This phase filter computationally mimics the function of a Shack-Hartmann wavefront sensor, although it may also be adapted to include a defocusing correction, as explained in the aforementioned article by N. D. Shemonski et al. A peak detection metric may be applied in conjunction with a guide-star based algorithm to iteratively fine-tune the aberration correction.

As another example, the correction algorithm 161 may take the form of the digital refocusing method in "Digital focusing of OCT images based on scalar diffraction theory and information entropy" by G. Liu et al., Biomed. Opt. Express 3, pp. 2774-2783 (2012).

The described digital refocusing method comprises taking the Fourier transform of the complex volumetric OCT data and rescaling it into linear k-space. This data is then resampled in the axial direction to obtain a sequence of enface frames along the axial direction. These enface frames are then digitally refocused onto a new focal plane by performing a search for a focal distance that minimises an entropy function of the image as it varies with each distance (such as a distance corresponding to the image with the minimum Shannon entropy out of a range of refocussed images at differing focal distances). Once all enface frames have been refocussed along the axial direction, the refocussed enface frames have an inverse Fourier transform applied to them such that refocussed image domain volumetric OCT data is obtained.

It should be noted, however, that the correction algorithm 161 is not limited to the examples set out above. The correction algorithm 161 may, for example, take the alternative form of one of the computational aberration correction algorithms, digital refocussing algorithms and interferometric synthetic aperture microscopy algorithms disclosed and/or referenced in "Computational optical coherence tomography [Invited]" by Y. Liu et al., Biomed. Opt. Express 8, pp. 1549-1574 (2017).

In the present example embodiment, where the line-field FD-OCT imaging system 100 is a line-field SS-OCT imaging system using the two-dimensional array of photodetector elements 400, the complex volumetric OCT data 170 generated by the OCT data processing hardware 160 based on the detection signal S_{d} may comprise a sequence of B-scans, and the corrected complex volumetric OCT data 180 generated by the OCT data processing hardware 160 may comprise a corresponding sequence of corrected B-scans. In this case, the OCT data processing hardware 160 may be arranged to generate each corrected B-scan of the corrected B-scans by executing, as the correction algorithm 161, a correction algorithm 161 (as described above) which uses phase information encoded in the corresponding B-scan (only). In other words, the correction algorithm 161 may generate each corrected B-scan of a plurality of corrected B-scans comprised in the corrected complex volumetric OCT data 180 by using the phase information encoded in a respective B-scan of the complex volumetric OCT data 170, which is based on a respective portion of the detection signal S_{d} that has been generated by the photodetector 140 in response to a detection of the interference line of light Lᵢ made by the photodetector 140 when the at least a segment of the line of light L_{L} was projected onto a respective location on a surface of the imaging target 130 during the scan (the respective portion of the complex volumetric OCT data 170 defining a B-scan corresponding to the corrected B-scan and at least one adjacent B-scan).

Where the line-field FD-OCT imaging system 100 is a line-field SS-OCT imaging system using the alternative one-dimensional array of photodetector elements 450, the OCT data processing hardware 160 may be arranged to generate each of the corrected B-scans by executing, as the correction algorithm 161, a correction algorithm 161 (as described above) which uses phase information encoded in the corresponding B-scan and at least one adjacent B-scan in the sequence of B-scans. In other words, the correction algorithm 161 may generate each corrected B-scan of a plurality of corrected B-scans comprised in the corrected complex volumetric OCT data 180 by using the phase information encoded in a portion of the complex volumetric OCT data 170, which is based on a portion of the detection signal S_{d} that has been generated by the photodetector 140 in response to a respective plurality of sequential detections of the interference line of light L_{I} made by the photodetector 140 when the at least a segment of the line of light L_{L} was projected during the scan onto different respective locations that are adjacent to each other on a surface of the imaging target 130 (the portion of the complex volumetric OCT data 170 defining a B-scan corresponding to the corrected B-scan and at least one B-scan that is adjacent to the B-scan).

Where the line-field FD-OCT imaging system 100 is a line-field SD-OCT imaging system, the photodetector comprises a two-dimensional spectrometer comprising a diffraction element and a two-dimensional array of photodetector elements as described above, and the correction algorithm 161 may take the form of a non-iterative deconvolution method, for example as described in the article titled "Complex Numerical Processing for In-Focus Line-Field Spectral-Domain Optical Coherence Tomography" by Y. Nakamura, Japanese Journal of Applied Physics Vol. 46 (2007): pp. 1774-1778.

In the described method, an inverse filter is multiplied by the Fourier transformed OCT image, with the resultant data being transformed into the real domain to obtain the deconvolved OCT image. The inverse filter is based on both an estimated point spread function for a perfect, aberration free version of the optical system used and the Fresnel diffraction light that diverges from the diffraction element to be detected by the 2D spectrometer.

The line-field FD-OCT imaging system 100 may, as in the present example embodiment, be arranged to measure a response of a retina of an eye to a light stimulus. Optoretinography provides an indication of how well the retina responds to light stimulation, and can provide a powerful tool for assessing the health of the eye. In optoretinogaphy, OCT data is acquired while the retina is being stimulated by a single light flash or two or more light flashes that are applied in sequence, typically over a period of two seconds or less. In some cases, the OCT data may be acquired at a high data rate while the retina is being stimulated by hundreds or thousands of light flashes over a period of 20-30 seconds, for example, and is then correlated with information defining the timing of applied light stimuli to provide an indication of the retinal response to the stimuli.

The line-field FD-OCT imaging system 100 may, as in the present example embodiment, comprise a light source 190 arranged to provide the light stimuli L_{S} to the eye, and a processor 195 arranged to process functional OCT image data, which has been acquired by the imaging system 100 scanning a retina of a subject while the retina is being repeatedly stimulated by a light stimulus from the light source 190, to generate an indication of a response of the retina to the light stimulus. In the present example embodiment, the processor 195 acquires, as the functional OCT image data, OCT image data 180 that has been generated by the imaging system 100 repeatedly imaging a region of the retina over a time period, and stimulus data defining a sequence of stimulus indicators each being indicative of a stimulation of the retina by the light source 190 in a respective time interval of a sequence of time intervals that spans the time period.

The processor 195 may, as in the present example embodiment, calculate a rolling window correlation between a sequence of enface images that is based on the OCT image data and stimulus indicators in the sequence of stimulus indicators, for example by: calculating, for each stimulus indicator, a product of the stimulus indicator and a respective windowed portion of the sequence of enface images comprising an enface image which is based on a portion of the OCT image data generated while the retina was being stimulated in accordance with the stimulus indicator; and combining the calculated products to generate the indication of the response of the retina to the light stimulus. Alternatively, the processor may calculate the rolling window correlation between the sequence of enface images and stimulus indicators in the sequence of stimulus indicators by calculating, for each stimulus indicator, a correlation between (i) stimulus indicators in a window comprising the stimulus indicator and a predetermined number of adjacent stimulus indicators, and (ii) enface images of the sequence of enface images that are based on a portion of the OCT image data generated while the retina was being stimulated in accordance with the stimulus indicators in the window, and the processor may further generate the indication of the response of the retina to the light stimulus by combining the calculated correlations.

Further details of how the processor 195 of the line-field FD-OCT imaging system 100 may process functional OCT image data, which has been acquired by line-field FD-OCT imaging system 100 imaging a retina while the retina is being repeatedly stimulated by a light stimulus, to generate an indication of a response of the retina to the light stimulus, may be obtained from US Patent No. 11,540,712.

The processing operations described in this patent with reference to B-scans of an acquired sequence of B-scans applies, mutatis mutandis, to the processing of enface images of an acquired sequence of enface images in the present example embodiment.

The line-field FD-OCT imaging system 100 may, however, be arranged to measure the response of the retina to a light stimulus by other techniques, for example using the approach to functional OCT described in the article titled "In vivo optical imaging of physiological responses to photostimulation in human photoreceptors" by D. Hillmann et al., Proceedings of the National Academy of Sciences, Vol. 113, No. 46, pages 13138-13143 (11 October 2016).

In general, the response of the retina to light stimuli may be measured by stimulating the retina with at least one flash of light, recording OCT scans of a portion of the retina before, during and (optionally) after the flash, and using the difference between the OCT scans (that are separated in time) to measure the response of the retina. A phase-sensitive approach may be used to measure the response of the retina in this way.

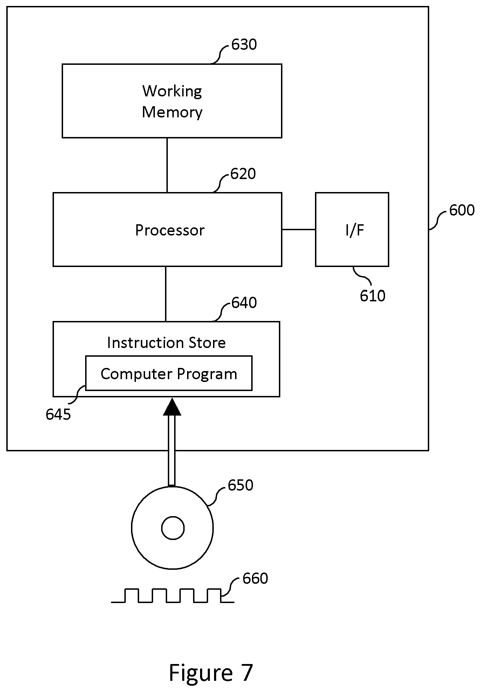

The OCT data processing hardware 160 and the processor 195 may be provided in any suitable form, for example as a programmable signal processing hardware 600 of the kind illustrated schematically in Figure 7. The programmable signal processing apparatus 600 comprises a communication interface (I/F) 610, for receiving the detection signal S_{d} from the photodetector 140, and outputting the corrected complex volumetric OCT data 180 and/or a graphical representation thereof (for example, in the form of an enface projection or one or more B-scans of the corrected complex volumetric OCT data 180) for displaying on a display, such a computer screen or the like. The signal processing hardware 600 further comprises a processor (e.g. a Central Processing Unit, CPU, and/or a Graphics Processing Unit, GPU) 620, a working memory 630 (e.g. a random-access memory) and an instruction store 640 storing a computer program 645 comprising the computer-readable instructions which, when executed by the processor 620, cause the processor 620 to perform various functions including those of the OCT data processing hardware 160 described herein. The working memory 630 stores information used by the processor 620 during execution of the computer program 645. The instruction store 640 may comprise a ROM (e.g. in the form of an electrically erasable programmable read-only memory (EEPROM) or flash memory) which is pre-loaded with the computer-readable instructions. Alternatively, the instruction store 640 may comprise a RAM or similar type of memory, and the computer-readable instructions of the computer program 645 can be input thereto from a computer program product, such as a non-transitory, computer-readable storage medium 650 in the form of a CD-ROM, DVDROM, etc. or a computer-readable signal 660 carrying the computer-readable instructions. In any case, the computer program 645, when executed by the processor 620, causes the processor 620 to perform the functions of the OCT data processing hardware 160 and optionally also of the processor 195 (functional OCT data processing hardware), as described herein. In other words, the OCT data processing hardware 160 of the example embodiment may comprise a computer processor 620 and a memory 640 storing computer-readable instructions which, when executed by the computer processor 620, cause the computer processor 620 to generate complex volumetric OCT data 170 of the imaging target 130 based on the detection signal S_{d} from the photodetector 140, wherein the complex volumetric OCT data 170 has an optical aberration, which may be observed in an enface projection and/or one or more B-scans of the complex volumetric OCT data 170. Further, the computer-readable instructions, when executed by the computer processor 620, cause the computer processor 620 to execute the correction algorithm 161 to remove at least some of the component from the complex volumetric OCT data 170 to produce corrected complex volumetric OCT data 180, as described above. Additionally, the processor 195 may comprise the computer processor 620 and the memory 640, which stores computer-readable instructions which, when executed by the computer processor 620, cause the computer processor 620 to process functional OCT image data, which has been acquired by the imaging system scanning a retina of a subject while the retina is being repeatedly stimulated by a light stimulus from the light source 190, to generate an indication of a response of the retina to the light stimulus.

It should be noted, however, that the OCT data processing hardware 160 (and/or the processor 195, where provided) may alternatively be implemented in non-programmable hardware, such as an ASIC, an FPGA or other integrated circuit dedicated to performing the functions of the OCT data processing hardware 160 described above (or the functions of the processor 195, as the case may be), or a combination of such non-programmable hardware and programmable hardware as described above with reference to Figure 7.

### [Second Example Embodiment]

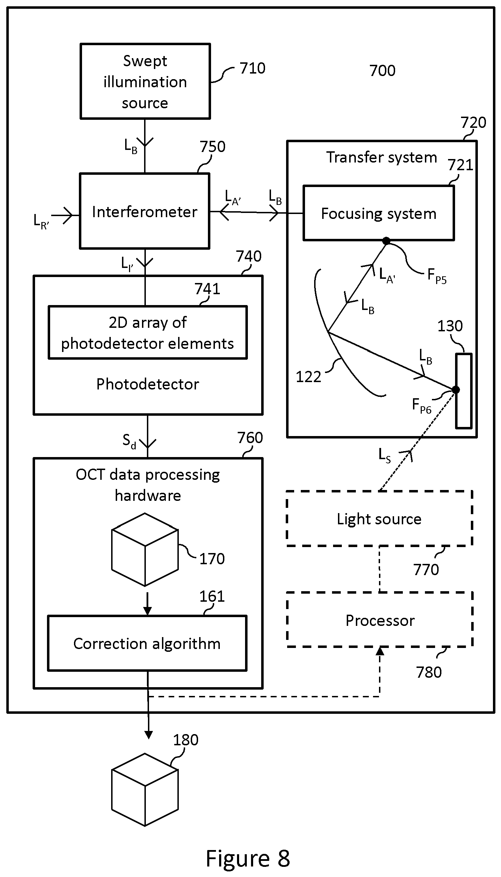

Figure 8 is a schematic illustration of a full-field SS-OCT imaging system 700 according to a second example embodiment herein. The full-field SS-OCT imaging system 700 may be a wide-field (WF) or an ultra-wide field (UWF) OCT imaging system, and comprises a swept illumination source 710, a transfer system 720, a photodetector 740, an interferometer 750 and OCT data processing hardware 760. Illustrated components and elements that are labelled with the same reference numerals as in the first example embodiment are the same and will not be described again.

The swept illumination source 710 is arranged to generate a beam of light L_{B} whose wavelength is swept over a range of values during imaging by the full-field swept-source OCT imaging system 700. For example, the swept illumination source 710 may, as in the present example embodiment, be a swept-source laser arranged to output substantially monochromatic light whose wavelength is swept over a range of wavelength values during the full-field scan.

The transfer system 720 comprises a curved mirror 722 having a first focal point F_{P5} and a conjugate second focal point F_{P6}. The curved mirror 722 may, as in the present example embodiment, be a parabolic mirror. However, curved mirror 722 may alternatively be an elliptical mirror, a spheroidal mirror, any reflective component having an aspherical reflective surface, such as a shape of a conical section like a parabola or hyperboloid, or may, more generally, have a shape described by one or more polynomial functions of two variables.

The transfer system 720 further comprises a focusing system 721, which is arranged to focus the beam of light L_{B} generated by the swept illumination source 710 at the first focal point F_{P5}. For example, the focusing system 721 may comprise at least one lens, which may include a cylindrical lens. The curved mirror 722 is arranged to guide the light beam L_{B} focused at the first focal point F_{P5} to the imaging target 130 located in a vicinity of the second focal point F_{P6} to provide full-field illumination of the imaging target 130 during imaging of the imaging target 130. The curved mirror 722 is further arranged to receive light which has been scattered by the imaging target 130 during the imaging of the imaging target 130 and aberrated by the curved mirror 722 to form an aberrated beam of light L_{A'} comprising at least one of a defocussing or distortion.

In ophthalmic applications, the curved mirror 722 may allow the full-field FD-OCT imaging system 700 to function as a WF OCT imaging system, by allowing the light beam L_{B} to illuminate a region of the fundus corresponding to an approximately 60- to 100-degree field of view, so as to capture the mid-periphery of the retina up to the posterior edge of the vortex vein ampulla. The illumination region could also be steered (e.g. with galvo mirrors) around a UWF imaging system to image different areas of the retina. Alternatively, the curved mirror 722 may allow the full-field FD-OCT imaging system 100 to function as a UWF OCT imaging system, by allowing the light beam L_{B} to illuminate a region of the fundus corresponding to an approximately 110- to 220-degree field of view, so as to capture the far periphery of the retina, including the anterior edge of the vortex vein ampulla and beyond.

The photodetector 740 comprises a two-dimensional array of photodetector elements 741. The two-dimensional array of photodetector elements 741 may, as in the present example embodiment, be a square grid of photodetector elements (i.e., a plurality of photodetector elements arranged in a grid of squares to form orthogonal rows and columns). However, the form of the array is not so limited, and may take any alternative form. The photodetector elements may, for example, be arranged in a rectangular grid or triangular grid instead. The photodetector elements may be photodiodes, phototransistors or capacitors of a charge-coupled device, for example.

The interferometer 750 is arranged to receive at least a portion of the aberrated return beam of light L_{A'} which has been received by the curved mirror 722 and to generate an interference light L_{I'} resulting from an interference between a reference light L_{R'} and at least a portion of the aberrated beam of light L_{A'}, and project (preferably the whole of) the interference light L_{I'} onto the two-dimensional array of photodetector elements 741. In other words, the reference light L_{R'} and the least a segment of the aberrated line of light L_{A'} received by the interferometer 750 from the transfer system 720 during the imaging are guided to coincide and interfere with one another, and the resulting interference light L_{I'} is directed to and received by the array of photodetector elements 741. The interferometer 750 may, as in the present example embodiment, be a Michelson interferometer using a beam-splitter to interfere the reference light L_{R'} and the at least a segment of the aberrated line of light L_{A'}. However, any free-space interferometer suitable for full-field OCT may be used, such as a Mach-Zehnder interferometer, for example.

Thus, the photodetector 740 is arranged to detect the interference light L_{I'} projected onto the two-dimensional array of photodetector elements 741 during imaging, and generate a detection signal S_{d} based on the detected interference light L_{I'}. The photodetector 740 generates the detection signal S_{d} by performing a photoelectric conversion of the interference light L_{I'} that is incident on the photodetector elements.

The photodetector elements of the two-dimensional array of photodetector elements 741 are arrayed in a first direction and in a second direction which is perpendicular to the first direction. The size of the two-dimensional array of photodetector elements 741 in the first and second direction, respectively, may, as in present example embodiment, be wider than a maximum size of the projection P_{I} of the interference light L_{I'} onto the two-dimensional array of photodetector elements 741 in the respective direction, and the projection P_{I} of the interference light L_{I'} onto the two-dimensional array of photodetector elements 741 is spanned in both of the directions by a plurality of the photodetector elements. The maximum size of the projection P_{I} may, as in the present example embodiment, be the displacement in a direction between the furthest point in the direction, at which light from the interference light L_{I'} is detectable by the photodetector elements (i.e. where the light intensity is above a minimum light intensity detectable by the photodetector elements), and the furthest point in an opposing direction at which light from the interference light L_{I'} is detectable by the photodetector elements (i.e. where the light intensity is above the minimum light intensity detectable by the photodetector elements). It is noted that the maximum size of the projection P_{I} may be defined in other ways, for example as the largest width out of the widths, in the second direction, of the interference light L_{I'} between locations along the first direction at which the intensity of the interference light L_{I'} has fallen to 0.1 %, 1 %, 5% or 10 % of the peak intensity value in the second direction at the respective location in the first direction (or the peak intensity value of the whole of the projected interference light L_{I'}).

The detection signal S_{d} generated by the photodetector 740 contains information about the defocussing and/or distortion (more specifically, information on how the measured intensity and phase are spatially distributed), which can be used by the correction algorithm 161 executed by the OCT data processing hardware 760 to digitally correct the defocussing and/or distortion in the acquired OCT data 170, as described below.

The OCT data processing hardware 760 is the same as the OCT data processing hardware 160, other than being adapted to process the detection signal S_{d} from a full-field rather than a line-field system. Once the complex volumetric OCT data 170 of the imaging target is generated, the correction algorithm 161 can be used to generate the corrected complex volumetric OCT data 180, as described in the first example embodiment. Generally, the correction algorithm 161 in the second example embodiment applies a phase filter to the complex volumetric OCT data 170 in the Fourier domain, before taking the inverse Fourier transform of the result. The phase filter is selected to reduce or remove phase errors which would produce the defocusing/distortion in an enface projection of the complex volumetric OCT data 170. An appropriate phase filter can be derived from a mathematical model of the full-field FD-OCT imaging system 700.

By way of further example to the correction algorithms described in the first example embodiment, the correction algorithm 161 may take the form of the aberration correction algorithm as described in the article titled "Aberration-free volumetric highspeed imaging of in vivo retina" by Hillmann et al., Sci Rep 6, 20 October 2016.

In the described aberration correction algorithm, an optimisation is performed of the Shannon entropy of a quantified measure of the image sharpness of a corrected image formed using a phase filter based on a parametrization, using Zernike polynomials, of an assumed phase-error wavefront. The optimisation finds the Zernike coefficients which minimise the Shannon entropy via a two-step approach of a simplex-downhill algorithm and, once near to the presumed global minimum, a gradient-based algorithm, such as a conjugate gradient method. A phase filter based on these optimised Zernike coefficients is used to form the corrected image.

It should be noted, however, that the correction algorithm 161 is not limited to the examples set out above or in the first example embodiment. The correction algorithm 161 may, for example, take the alternative form of one of the defocus correction algorithms, such as the forward model, inverse scattering or the digital adaptive optics based on sub aperture correlation algorithms, disclosed and/or referenced in "Numerical focusing methods for full field OCT: a comparison based on a common signal model" by A. Kumar et al., Opt. Express 22, pp. 16061-16078 (2014).

The full-field SS-OCT imaging system 700 may, as in the present example embodiment, comprise a light source 770 and a processor 780 which have the same structure and functionality as the light source 190 and the processor 195 of the first example embodiment.

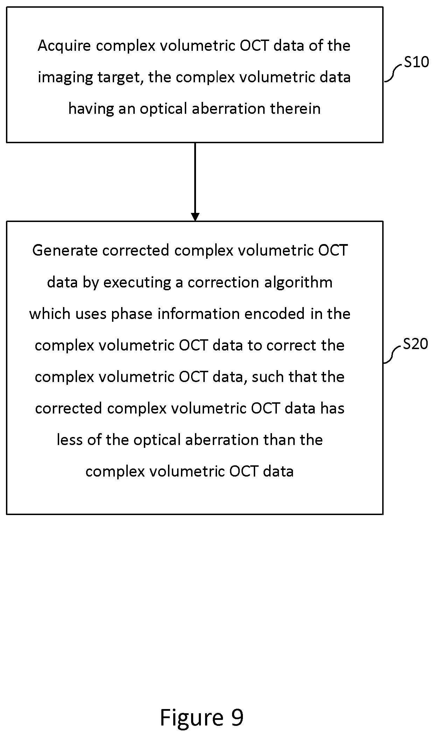

Figure 9 is a flow diagram illustrating a process (complex volumetric OCT data correction algorithm) by which the OCT data processing hardware 160, 760 of either the first example embodiment or the second example embodiment generates the corrected complex volumetric OCT data 180 by executing the correction algorithm 161, which processes phase information encoded in the complex volumetric OCT data to correct the complex volumetric OCT data.

In process S10 of Figure 9, the OCT data processing hardware 160, 760 acquires the complex volumetric OCT data of the imaging target 130 that has an aberration therein and has been generated by the line-scan Fourier-domain OCT imaging system 100 or the full-field Fourier-domain OCT imaging system 700 (as the case may be), for example by receiving this data from the OCT data processing hardware 160, 760, e.g. by retrieving the data from a memory (e.g. working memory 630 shown in Figure 7) of the OCT data processing hardware 160, 760.

Then, in process S20 of Figure 9, the OCT data processing hardware 160, 760 generates the corrected complex volumetric OCT data 180 by executing the correction algorithm 161 described above.

The process of Figure 9 may be performed by OCT data processing hardware 160, 760 implemented in the form of a programmable signal processing hardware 600 as described above with reference to Figure 7, which operates in accordance with instructions included in a complex volumetric OCT data correction computer program when the computer program is executed by one or more processors. This computer program may be stored in a computer program product, such as a non-transitory, computer-readable storage medium in the form of a CD-ROM, DVDROM, etc. or a computer-readable signal carrying the computer-readable instructions.

The complex volumetric OCT data correction computer program may form part of a computer program which also generates the complex volumetric OCT data of the imaging target 130 based on the detection signal S_{d}, or it may alternatively be a separate program, which may or may not be executed by the same one or more processors that generate the complex volumetric OCT data of the imaging target 130 based on the detection signal S_{d}. In implementations where the complex volumetric OCT data correction computer program is executed by a first set of one or more processors, while the complex volumetric OCT data of the imaging target 130 is generated on the basis of the detection signal S_{d} by a second set of (different) one or more processors, the first set of one or more processors may acquire the complex volumetric OCT data of the imaging target 130 in process S10 of Figure 9 by receiving this data from the second set of one or more processors via appropriate interfaces between the two sets of processors.

The computer-implemented method described above with reference to Figure 9 may form part of a method of processing complex volumetric OCT data of an imaging target 130 generated by either the line-scan Fourier-domain OCT imaging system 100 of the first example embodiment or the full-field Fourier-domain OCT imaging system 700 of the second example embodiment, which includes, prior to the acquisition of the complex volumetric OCT data of the imaging target 130 in process S10 of Figure 9, the line-scan Fourier-domain OCT imaging system 100 (or the full-field Fourier-domain OCT imaging system 700, as the case may be) imaging the imaging target 130 to generate the complex volumetric OCT data of the imaging target 130. In other words, in a variant of the method of Figure 9, which is not implemented entirely by a computer, process S10 may comprise acquiring the complex OCT data of the imaging target 130 by imaging the imaging target 130 to generate the complex volumetric OCT data, using the line-scan Fourier-domain OCT imaging system 100 (or the full-field Fourier-domain OCT imaging system 700, as the case may be).

In the foregoing description, example aspects are described with reference to several example embodiments. Accordingly, the specification should be regarded as illustrative, rather than restrictive. Similarly, the figures illustrated in the drawings, which highlight the functionality and advantages of the example embodiments, are presented for example purposes only. The architecture of the example embodiments is sufficiently flexible and configurable, such that it may be utilized in ways other than those shown in the accompanying figures.

Some aspects of the examples presented herein, such as the function of the controller, may be provided as a computer program, or software, such as one or more programs having instructions or sequences of instructions, included or stored in an article of manufacture such as a machine-accessible or machine-readable medium, an instruction store, or computer-readable storage device, each of which can be non-transitory, in one example embodiment. The program or instructions on the non-transitory machine-accessible medium, machine-readable medium, instruction store, or computer-readable storage device, may be used to program a computer system or other electronic device. The machine- or computer-readable medium, instruction store, and storage device may include, but are not limited to, floppy diskettes, optical disks, and magneto-optical disks or other types of media/machine-readable medium/instruction store/storage device suitable for storing or transmitting electronic instructions. The techniques described herein are not limited to any particular software configuration. They may find applicability in any computing or processing environment. The terms "computer-readable", "machine-accessible medium", "machine-readable medium", "instruction store", and "computer-readable storage device" used herein shall include any medium that is capable of storing, encoding, or transmitting instructions or a sequence of instructions for execution by the machine, computer, or computer processor and that causes the machine/computer/computer processor to perform any one of the methods described herein. Furthermore, it is common in the art to speak of software, in one form or another (e.g., program, procedure, process, application, module, unit, logic, and so on), as taking an action or causing a result. Such expressions are merely a shorthand way of stating that the execution of the software by a processing system causes the processor to perform an action to produce a result.

Some or all of the functionality of the controller may also be implemented by the preparation of application-specific integrated circuits, field-programmable gate arrays, or by interconnecting an appropriate network of conventional component circuits.

A computer program product may be provided in the form of a storage medium or media, instruction store(s), or storage device(s), having instructions stored thereon or therein which can be used to control, or cause, a computer or computer processor to perform any of the procedures of the example embodiments described herein. The storage medium/instruction store/storage device may include, by example and without limitation, an optical disc, a ROM, a RAM, an EPROM, an EEPROM, a DRAM, a VRAM, a flash memory, a flash card, a magnetic card, an optical card, nanosystems, a molecular memory integrated circuit, a RAID, remote data storage/archive/warehousing, and/or any other type of device suitable for storing instructions and/or data.

Stored on any one of the computer-readable medium or media, instruction store(s), or storage device(s), some implementations include software for controlling both the hardware of the system and for enabling the system or microprocessor to interact with a human user or other mechanism utilizing the results of the example embodiments described herein. Such software may include without limitation device drivers, operating systems, and user applications. Ultimately, such computer-readable media or storage device(s) further include software for performing example aspects of the invention, as described above.

Included in the programming and/or software of the system are software modules for implementing the procedures described herein. In some example embodiments herein, a module includes software, although in other example embodiments herein, a module includes hardware, or a combination of hardware and software.

While various example embodiments of the present invention have been described above, it should be understood that they have been presented by way of example, and not limitation. It will be apparent to persons skilled in the relevant art(s) that various changes in form and detail can be made therein. Thus, the present invention should not be limited by any of the above-described example embodiments, but should be defined only in accordance with the following claims and their equivalents.

Further, the purpose of the Abstract is to enable the Patent Office and the public generally, and especially the scientists, engineers and practitioners in the art who are not familiar with patent or legal terms or phraseology, to determine quickly from a cursory inspection the nature and essence of the technical disclosure of the application. The Abstract is not intended to be limiting as to the scope of the example embodiments presented herein in any way. It is also to be understood that any procedures recited in the claims need not be performed in the order presented.

While this specification contains many specific embodiment details, these should not be construed as limitations on the scope of any inventions as defined by the claims, but rather as descriptions of features specific to particular embodiments described herein. Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In certain circumstances, multitasking and parallel processing may be advantageous. Moreover, the separation of various components in the embodiments described above should not be understood as requiring such separation in all embodiments, and it should be understood that the described program components and systems can generally be integrated together in a single software product or packaged into multiple software products.

Having now described some illustrative embodiments and embodiments, it is apparent that the foregoing is illustrative and not limiting, having been presented by way of example. In particular, although many of the examples presented herein involve specific combinations of apparatus or software elements, those elements may be combined in other ways to accomplish the same objectives. Acts, elements and features discussed only in connection with one embodiment are not intended to be excluded from a similar role in other embodiments or embodiments.

## Claims

1. A line-field Fourier-domain optical coherence tomography, OCT, imaging system (100), comprising:
a line-field illumination source (110) arranged to generate a line of light (L_{L});
a scanning system (120) comprising a scanning element (121) and a curved mirror (122) having a first focal point (F_{P1}) and a conjugate second focal point (F_{P2}), the scanning element (121) being located at the first focal point (F_{P1}) and arranged to perform a scan of an imaging target (130) via the second focal point (F_{P2}), by scanning at least a segment of the line of light (L_{L}) across the imaging target (130) via the curved mirror (122), the scanning element (121) being further arranged to receive, via the curved mirror (122), light which has been scattered by the imaging target (130) during the scan and aberrated by the curved mirror (122) to form an aberrated line of light (L_{A}) comprising at least one of a defocussing or a distortion;
a photodetector (140) comprising an array of photodetector elements (141);
an interferometer (150) arranged to receive at least a segment of the aberrated line of light (L_{A}) via the scanning element (121), generate an interference line of light (L_{I}) resulting from an interference between a reference light (L_{R}) and the at least a segment of the aberrated line of light (L_{A}) received via the scanning element (121), and project the interference line of light (L_{I}) onto the array of photodetector elements (141),
wherein the photodetector (140) is arranged to detect the interference line of light (L_{I}) projected onto the array of photodetector elements (141) during the scan, and generate a detection signal (S_{d}) based on the detected interference line of light (L_{I}); and
OCT data processing hardware (160) arranged to:
generate complex volumetric OCT data (170) of the imaging target (130) based on the detection signal (S_{d}), wherein the complex volumetric OCT data (170) has an optical aberration therein; and
generate corrected complex volumetric OCT data (180) by executing a correction algorithm (161) which uses phase information encoded in the complex volumetric OCT data (170) to correct the complex volumetric OCT data (170), such that the corrected complex volumetric OCT data (180) has less of the optical aberration than the complex volumetric OCT data (170).

2. The line-field Fourier-domain OCT imaging system (100) according to Claim 1, wherein
the line-field Fourier-domain OCT imaging system is a line-field swept-source OCT imaging system,
the line-field illumination source (110) is a swept line-field illumination source arranged to generate the line of light, and
the array of photodetector elements (141) is a one-dimensional array of photodetector elements, and the photodetector elements of the one-dimensional array of photodetector elements are arrayed along a length of a projection of the interference line of light onto the one-dimensional array of photodetector elements.

3. The line-field Fourier-domain OCT imaging system (100) according to Claim 2, wherein the photodetector elements of the one-dimensional array have a width, in a direction perpendicular to a length of the projection of the interference line of light onto the one-dimensional array of photodetector elements, which is wider than a maximum width of the projection of the interference line of light onto the one-dimensional array of photodetector elements.

4. The line-field Fourier-domain OCT imaging system (100) according to Claim 1, wherein
the line-field Fourier-domain OCT imaging system is a line-field swept-source OCT imaging system,
the line-field illumination source (110) is a swept line-field illumination source, and the array of photodetector elements (141) is a two-dimensional array of photodetector elements, and the photodetector elements of the two-dimensional array of photodetector elements are arrayed in a first direction along a length of a projection of the interference line of light onto the two-dimensional array of photodetector elements, and in a second direction which is perpendicular to the first direction.

5. The line-field Fourier-domain OCT imaging system (100) according to Claim 4, wherein
a width of the two-dimensional array of photodetector elements, in the second direction, is wider than a maximum width of the projection of the interference line of light onto the two-dimensional array of photodetector elements, and
the projection of the interference line of light onto the two-dimensional array of photodetector elements is spanned in the second direction by a plurality of the photodetector elements.

6. The line-field Fourier-domain OCT imaging system (100) according to Claim 1, wherein
the line-field Fourier-domain OCT imaging system (100) is a line-field spectral domain OCT imaging system,
the line-field illumination source (110) is a broadband line-field illumination source arranged to generate the line of light (L_{L}),
the array of photodetector elements (141) is a two-dimensional array of photodetector elements,
and the photodetector (140) comprises a two-dimensional spectrometer comprising a diffraction element and the two-dimensional array of photodetector elements, the diffraction element being arranged to disperse a spectral content of the interference line of light across the two-dimensional array of photodetector elements in a first direction, which is perpendicular to a second direction along which a projection of the interference line of light onto the one-dimensional array of photodetector elements extends, wherein the photodetector elements of the two-dimensional array are arrayed in the first direction and in the second direction.

7. The line-field Fourier-domain OCT imaging system (100) according to any preceding claim, wherein
the scanning element (121) is a first scanning element arranged to scan the at least a segment of the line of light (L_{L}) in a first direction across a surface of the imaging target (130) such that projections of the at least a segment of the line of light (L_{L}) onto the surface of the imaging target (130) during the scan are displaced relative to each other along the first direction, and
the scanning system (120) further comprises a second scanning element, which is arranged to reflect the at least a segment of the line of light (L_{L}) from the line-field illumination source (110) toward the first scanning element (121), and arranged to change, in a second direction that is perpendicular to the first direction, and in a third direction opposite to the second direction, a position at which the first scanning element (121) is to scan the at least a segment of the line of light (L_{L}) across the surface of the imaging target (130).

8. The line-field Fourier-domain OCT imaging system (100) according to Claim 7, wherein the first scanning element (121) is arranged to scan the at least a segment of the line of light (L_{L}) in the first direction across the surface of the imaging target (130), or in a direction opposite to the first direction across the surface of the imaging target (130), both before and after the change.

9. The line-field Fourier-domain OCT imaging system (100) according to any preceding claim, wherein the curved mirror (122) is a spheroid mirror having an axis of circular symmetry, and the scanning element (121) is arranged to perform the scan of the imaging target (130) by scanning the at least a segment of the line of light (L_{L}) across the imaging target (130) via the spheroid mirror such that the at least a segment of the line of light (L_{L}) incident on the spheroid mirror propagates in a plane which is parallel to the axis of circular symmetry of the spheroid mirror.

10. The line-field Fourier-domain OCT imaging system (100) of any of Claims 1 to 8, wherein
the curved mirror (122) is a spheroid mirror, and
the scanning element (121) is arranged to perform the scan of the imaging target (130) by scanning the at least a segment of the line of light (L_{L}) across the imaging target (130) via the spheroid mirror such that a portion of the spheroid mirror, onto which the at least a segment of the line of light is projected, has reflective symmetry about a plane parallel to and passing through the axis of circular symmetry.

11. The line-field Fourier-domain OCT imaging system (100) of any preceding claim, further comprising a mask (800) having a first region (801) and a second region (802) that surrounds the first region (801), wherein the first region (801) has a higher transparency than the second region (802), the mask (800) both being located in the line-field Fourier-domain OCT imaging system (100) and having the first region (801) shaped so as to allow at least some of the interference line of light (L_{I}) to propagate to the array of photodetector elements (141) and at least partially prevent light other than the interference line of light (L_{I}) from propagating to the array of photodetector elements (141).

12. The line-field Fourier-domain OCT imaging system (100) of any preceding claim, further comprising a light source (190), wherein the line-field Fourier-domain OCT imaging system (100) is arranged to measure a response of a retina of an eye to a light stimulus generated by the light source (190).

13. A full-field swept-source optical coherence tomography, OCT, imaging system (700), comprising:
a swept illumination source (710) arranged to generate a beam of light (L_{B}) whose wavelength is swept over a range of values during imaging by the full-field swept-source OCT imaging system (700);
a transfer system (720) comprising a curved mirror (722) having a first focal point (F_{P1}) and a conjugate second focal point (F_{P2}), the transfer system (720) further comprising a focusing system (721) which is arranged to focus the beam of light (L_{B}) generated by the swept illumination source (710) at the first focal point (F_{P1}), the curved mirror (722) being arranged to guide the light beam (L_{B}) focused at the first focal point (F_{P1}) to an imaging target (130) via the second focal point (F_{P2}) so as to provide full-field illumination of the imaging target (130) during imaging of the imaging target (130), the curved mirror (722) being further arranged to receive light which has been scattered by the imaging target (130) during the imaging of the imaging target (130) and aberrated by the curved mirror (722) to form an aberrated beam of light (L_{A}) comprising at least one of a defocussing or distortion;
a photodetector (740) comprising a two-dimensional array of photodetector elements (741);
an interferometer (750) arranged to receive at least a portion of the light which has been received by the curved mirror (722) and to generate an interference light (L_{I}) resulting from an interference between a reference light (L_{R}) and the at least a portion of the aberrated beam of light (L_{A}), and project the interference light (L_{I}) onto the two-dimensional array of photodetector elements (741),
wherein the photodetector (740) is arranged to detect the interference light (L_{I}) projected onto the two-dimensional array of photodetector elements (741) during the imaging, and generate a detection signal (S_{d}) based on the detected interference light (L_{I}); and
OCT data processing hardware (760) arranged to:
generate complex volumetric OCT data (170) of the imaging target (130) based on the detection signal (S_{d}), wherein the complex volumetric OCT data (170) has an aberration therein; and
generate corrected complex volumetric OCT data (180) by executing a correction algorithm (161) which uses phase information encoded in the complex volumetric OCT data (170) to correct the complex volumetric OCT data (170), such that the corrected complex volumetric OCT data (180) has less of the aberration than the complex volumetric OCT data (170).

14. The full-field swept-source OCT imaging system (700) of Claim 13, further comprising a light source (770), wherein the full-field swept-source OCT imaging system (700) is arranged to measure a response of a retina of an eye to a light stimulus generated by the light source (770).

15. A computer-implemented method of processing complex volumetric OCT data of an imaging target (130) generated by either the line-scan Fourier-domain OCT imaging system (100) of Claim 1 or the full-field Fourier-domain OCT imaging system (700) of Claim 13, the method comprising:
acquiring (S10) the complex volumetric OCT data (170) of the imaging target (130), the complex volumetric OCT data (170) having an optical aberration therein; and
generating (S20) corrected complex volumetric OCT data by executing a correction algorithm which uses phase information encoded in the complex volumetric OCT data to correct the complex volumetric OCT data, such that the corrected complex volumetric OCT data (180) has less of the optical aberration than the complex volumetric OCT data (170).

## Patentansprüche

1. Bildgebungssystem (100) für optische Linienfeld-Fourier-Domänen-Kohärenztomographie (OCT), umfassend:
eine Linienfeldbeleuchtungsquelle (110), die so angeordnet ist, dass sie eine Lichtlinie (L_{L}) erzeugt;
ein Abtastsystem (120), das ein Abtastelement (121) und einen gekrümmten Spiegel (122) mit einem ersten Brennpunkt (F_{P1}) und einem konjugierten zweiten Brennpunkt (F_{P2}) umfasst, wobei das Abtastelement (121) an dem ersten Brennpunkt (F_{P1}) angeordnet ist und so eingerichtet ist, dass es eine Abtastung eines Abbildungsziels (130) über den zweiten Brennpunkt (F_{P2}) durchführt, indem mindestens ein Segment der Lichtlinie (L_{L}) über das Abbildungsziel (130) über den gekrümmten Spiegel (122) abgetastet wird, wobei das Abtastelement (121) ferner so angeordnet ist, dass es über den gekrümmten Spiegel (122) Licht empfängt, das von dem Abbildungsziel (130) während der Abtastung gestreut und durch den gekrümmten Spiegel (122) aberriert wurde, um eine aberrierte Lichtlinie (L_{A}) zu bilden, die eine Defokussierung oder/und eine Verzerrung umfasst; einen Fotodetektor (140), der eine Anordnung von Fotodetektorelementen (141) umfasst;
ein Interferometer (150), das ausgelegt ist, um mindestens ein Segment der aberrierten Lichtlinie (L_{A}) über das Abtastelement (121) zu empfangen, eine Interferenzlichtlinie (L_{I}) zu erzeugen, die aus einer Interferenz zwischen einem Referenzlicht (L_{R}) und dem mindestens einen Segment der aberrierten Lichtlinie (L_{A}) resultiert, das über das Abtastelement (121) empfangen wird, und die Interferenzlichtlinie (L_{I}) auf die Anordnung von Fotodetektorelementen (141) zu projizieren,
wobei der Fotodetektor (140) so angeordnet ist, dass er die Interferenzlinie des Lichts (L_{I}) detektiert, die während des - Scans auf die Anordnung von Fotodetektorelementen (141) projiziert wird, und ein Detektionssignal (S_{d}) auf der Grundlage der detektierten Interferenzlinie des Lichts (L_{I}) erzeugt; und
OCT-Datenverarbeitungshardware (160), die so angeordnet ist, dass sie:
komplexe volumetrische OCT-Daten (170) des Abbildungsziels (130) auf der Grundlage des Erfassungssignals (S_{d}) zu erzeugen, wobei die komplexen volumetrischen OCT-Daten (170) eine optische Aberration enthalten; und
korrigierte komplexe volumetrische OCT-Daten (180) durch Ausführen eines Korrekturalgorithmus (161), der in den komplexen volumetrischen OCT-Daten (170) kodierte Phaseninformation verwendet, zu erzeugen, um die komplexen volumetrischen OCT-Daten (170) zu korrigieren, so dass die korrigierten komplexen volumetrischen OCT-Daten (180) weniger von der optischen Aberration aufweisen als die komplexen volumetrischen OCT-Daten (170).

2. Linienfeld-Fourier-Domänen-OCT-Bildgebungssystem (100) gemäß Anspruch 1, wobei
das Linienfeld-Fourier-Domänen-OCT-Bildgebungssystem ein Linienfeld-Swept-Source-OCT-Bildgebungssystem ist,
die Linienfeld-Beleuchtungsquelle (110) eine geschwungene Linienfeld-Beleuchtungsquelle ist, die ausgelegt ist, die Lichtlinie zu erzeugen, und
die Anordnung von Fotodetektorelementen (141) eine eindimensionale Anordnung von Fotodetektorelementen ist, und die Fotodetektorelemente des eindimensionalen Arrays von Fotodetektorelementen entlang einer Länge einer Projektion der Interferenzlinie von Licht auf die eindimensionale Anordnung von Fotodetektorelementen angeordnet sind.

3. Linienfeld-Fourier-Domänen-OCT-Bildgebungssystem (100) gemäß Anspruch 2, wobei die Fotodetektorelemente der eindimensionalen Anordnung in einer Richtung senkrecht zu einer Länge der Projektion der Interferenzlinie des Lichts auf die eindimensionale Anordnung von Fotodetektorelementen eine Breite aufweisen, die breiter ist als eine maximale Breite der Projektion der Interferenzlinie des Lichts auf die eindimensionale Anordnung von Fotodetektorelementen.

4. Linienfeld-Fourier-Domänen-OCT-Bildgebungssystem (100) gemäß Anspruch 1, wobei
das Linienfeld-Fourier-Domänen-OCT-Bildgebungssystem ein Linienfeld-Swept-Source-OCT-Bildgebungssystem ist,
die Linienfeld-Beleuchtungsquelle (110) eine Linienfeld-Beleuchtungs-Swept-Source ist, und
die Anordnung von Fotodetektorelementen (141) eine zweidimensionale Anordnung von Fotodetektorelementen ist, und die Fotodetektorelemente der zweidimensionalen Anordnung von Fotodetektorelementen in einer ersten Richtung entlang einer Länge einer Projektion der Interferenzlinie von Licht auf die zweidimensionale Anordnung von Fotodetektorelementen und in einer zweiten Richtung, die senkrecht zu der ersten Richtung ist, angeordnet sind.

5. Linienfeld-Fourier-Domänen-OCT-Bildgebungssystem (100) gemäß Anspruch 4, wobei
eine Breite der zweidimensionalen Anordnung von Fotodetektorelementen in der zweiten Richtung breiter ist als eine maximale Breite der Projektion der Interferenzlinie des Lichts auf die zweidimensionale Anordnung von Fotodetektorelementen, und
die Projektion der Interferenzlinie von Licht auf die zweidimensionale Anordnung von Fotodetektorelementen in der zweiten Richtung von einer Vielzahl der Fotodetektorelemente aufgespannt wird.

6. Linienfeld-Fourier-Domänen-OCT-Bildgebungssystem (100) gemäß Anspruch 1, wobei
das Linienfeld-Fourier-Domänen-OCT-Bildgebungssystem (100) ist ein Linienfeld-OCT-Bildgebungssystem im Spektralbereich,
die Linienfeldbeleuchtungsquelle (110) eine breitbandige Linienfeldbeleuchtungsquelle ist, die so angeordnet ist, dass sie die Lichtlinie (L_{L}) erzeugt,
die Anordnung von Fotodetektorelementen (141) eine zweidimensionale Anordnung von Fotodetektorelementen ist,
und der Fotodetektor (140) ein zweidimensionales Spektrometer umfasst, das ein Beugungselement und die zweidimensionale Anordnung von Fotodetektorelementen umfasst, wobei das Beugungselement so angeordnet ist, dass es einen Spektralinhalt der Interferenzlinie des Lichts über die zweidimensionale Anordnung von Fotodetektorelementen in einer ersten Richtung streut, die senkrecht zu einer zweiten Richtung ist, entlang der sich eine Projektion der Interferenzlinie des Lichts auf die eindimensionale Anordnung von Fotodetektorelementen erstreckt, wobei die Fotodetektorelemente der zweidimensionalen Anordnung in der ersten Richtung und in der zweiten Richtung angeordnet sind.

7. Linienfeld-Fourier-Domänen-OCT-Bildgebungssystem (100) gemäß einem der vorhergehenden Ansprüche, wobei das Abtastelement (121) ein erstes Abtastelement ist, das so angeordnet ist, dass es das mindestens eine Segment der Lichtlinie (L_{L}) in einer ersten Richtung über eine Oberfläche des Abbildungsziels (130) abtastet, so dass Projektionen des mindestens einen Segments der Lichtlinie (L_{L}) auf die Oberfläche des Abbildungsziels (130) während des Abtastens relativ zueinander entlang der ersten Richtung verschoben werden, und
das Abtastsystem (120) ferner ein zweites Abtastelement umfasst, das so angeordnet ist, dass es das mindestens eine Segment der Lichtlinie (L_{L}) von der Linienfeldbeleuchtungsquelle (110) zu dem ersten Abtastelement (121) reflektiert, und so angeordnet ist, dass es in einer zweiten Richtung, die senkrecht zu der ersten Richtung ist, und in einer dritten Richtung, die der zweiten Richtung entgegengesetzt ist, eine Position ändert, an der das erste Abtastelement (121) das mindestens eine Segment der Lichtlinie (L_{L}) über die Oberfläche des Abbildungsziels (130) abtasten soll.

8. Linienfeld-Fourier-Domänen-OCT-Abbildungssystem (100) gemäß Anspruch 7, wobei das erste Abtastelement (121) so angeordnet ist, dass es das mindestens eine Segment der Lichtlinie (L_{L}) in der ersten Richtung über die Oberfläche des Abbildungsziels (130) oder in einer der ersten Richtung entgegengesetzten Richtung über die Oberfläche des Abbildungsziels (130) sowohl vor als auch nach der Änderung abtastet.

9. Linienfeld-Fourier-Domänen-OCT-Abbildungssystem (100) gemäß einem der vorhergehenden Ansprüche, wobei der gekrümmte Spiegel (122) ein Sphäroidspiegel mit einer kreisförmigen Symmetrieachse ist, und das Abtastelement (121) so angeordnet ist, dass es die Abtastung des Abbildungsziels (130) durch Abtasten des mindestens einen Segments der Lichtlinie (L_{L}) über das Abbildungsziel (130) über den Sphäroidspiegel durchführt, so dass sich das mindestens eine Segment der Lichtlinie (L_{L}), das auf den Sphäroidspiegel auftrifft, in einer Ebene ausbreitet, die parallel zu der Achse der kreisförmigen Symmetrie des Sphäroidspiegels ist.

10. Linienfeld-Fourier-Domänen-OCT-Bildgebungssystem (100) gemäß einem der Ansprüche 1 bis 8, wobei
der gekrümmte Spiegel (122) ein Sphäroidspiegel ist, und
das Abtastelement (121) so angeordnet ist, dass es die Abtastung des Abbildungsziels (130) durch Abtasten des mindestens einen Segments der Lichtlinie (L_{L}) über das Abbildungsziel (130) über den Sphäroidspiegel so durchführt, dass ein Teil des Sphäroidspiegels, auf den das mindestens eine Segment der Lichtlinie projiziert wird, eine Reflexionssymmetrie um eine Ebene aufweist, die parallel zur und durch die Achse der kreisförmigen Symmetrie verläuft.

11. Linienfeld-Fourier-Domänen-OCT-Bildgebungssystem (100) gemäß einem der vorhergehenden Ansprüche, das ferner eine Maske (800) mit einem ersten Bereich (801) und einem zweiten Bereich (802), der den ersten Bereich (801) umgibt, umfasst, wobei der erste Bereich (801) eine höhere Transparenz als der zweite Bereich (802) aufweist, wobei die Maske (800) sowohl in dem Linienfeld-Fourier-Domänen-OCT-Bildgebungssystem (100) angeordnet ist als auch den ersten Bereich (801) aufweist, der so geformt ist, dass er es zumindest einem Teil der Interferenzlinie des Lichts (L_{I}) ermöglicht, sich zu der Anordnung von Fotodetektorelementen (141) auszubreiten, und zumindest teilweise verhindert, dass sich anderes Licht als die Interferenzlinie des Lichts (L_{I}) zu der Anordnung von Fotodetektorelementen (141) ausbreitet.

12. Linienfeld-Fourier-Domänen-OCT-Bildgebungssystem (100) gemäß einem der vorhergehenden Ansprüche, das ferner eine Lichtquelle (190) umfasst, wobei das Linienfeld-Fourier-Domänen-OCT-Bildgebungssystem (100) so angeordnet ist, dass es eine Reaktion einer Netzhaut eines Auges auf einen von der Lichtquelle (190) erzeugten Lichtreiz misst.

13. Bildgebungssystem (700) für optische Vollfeld-Swept-Source-Kohärenztomographie (OCT), umfassend:
eine Swept-Beleuchtungsquelle (710), die so angeordnet ist, dass sie einen Lichtstrahl (L_{B}) erzeugt, dessen Wellenlänge während der Bildgebung durch das Vollfeld-Swept-Source-OCT-Bildgebungssystem (700) über einen Bereich von Werten ge-swept wird;
ein Übertragungssystem (720), das einen gekrümmten Spiegel (722) mit einem ersten Brennpunkt (F_{P1}) und einem konjugierten zweiten Brennpunkt (F_{P2}) umfasst, wobei das Übertragungssystem (720) ferner ein Fokussierungssystem (721) umfasst, das so ausgelegt ist, dass es den von der überstrichenen Beleuchtungsquelle (710) erzeugten Lichtstrahl (L_{B}) auf den ersten Brennpunkt (F_{P1}) fokussiert, der gekrümmte Spiegel (722) so angeordnet ist, dass er den Lichtstrahl (L_{B}), der am ersten Brennpunkt (F_{P1}) fokussiert ist, über den zweiten Brennpunkt (F_{P2}) zu einem Abbildungsziel (130) leitet, um eine Vollfeldbeleuchtung des Abbildungsziels (130) während der Bildgebung des Abbildungsziels (130) bereitzustellen, der gekrümmte Spiegel (722) ferner so ausgelegt ist, dass er Licht empfängt, das durch das Abbildungsziel (130) während der Bildgebung des Abbildungsziels (130) gestreut und durch den gekrümmten Spiegel (722) aberriert wurde, um einen aberrierten Lichtstrahl (L_{A}) zu bilden, der mindestens eine Defokussierung oder Verzerrung umfasst;
einen Fotodetektor (740), der eine zweidimensionale Anordnung von Fotodetektorelementen (741) umfasst;
ein Interferometer (750), das so ausgelegt ist, dass es zumindest einen Teil des Lichts empfängt, das von dem gekrümmten Spiegel (722) empfangen wurde, und ein Interferenzlicht (L_{I}) erzeugt, das aus einer Interferenz zwischen einem Referenzlicht (L_{R}) und dem zumindest einen Teil des aberrierten Lichtstrahls (L_{A}) resultiert, und das Interferenzlicht (L_{I}) auf die zweidimensionale Anordnung von Fotodetektorelementen (741) projiziert,
wobei der Fotodetektor (740) ausgelegt ist, um das Interferenzlicht (L_{I}), das während der Bildgebung auf die zweidimensionale Anordnung von Fotodetektorelementen (741) projiziert wird, zu detektieren und ein Detektionssignal (S_{d}) basierend auf dem detektierten Interferenzlicht (L_{I}) zu erzeugen; und
OCT-Datenverarbeitungshardware (760), die ausgelegt ist:
komplexe volumetrische OCT-Daten (170) des Abbildungsziels (130) auf der Basis des Detektionssignals (S_{d}) zu erzeugen, wobei die komplexen volumetrischen OCT-Daten (170) eine optische Aberration in sich aufweisen; und
korrigierte komplexe volumetrische OCT-Daten (180) durch Ausführen eines Korrekturalgorithmus (161), der in den komplexen volumetrischen OCT-Daten (170) kodierte Phaseninformation verwendet, zu erzeugen, um die komplexen volumetrischen OCT-Daten (170) zu korrigieren, so dass die korrigierten komplexen volumetrischen OCT-Daten (180) weniger von der optischen Aberration aufweisen als die komplexen volumetrischen OCT-Daten (170).

14. Vollfeld-Swept-Source-OCT-Abbildungssystem (700) gemäß Anspruch 13, das ferner eine Lichtquelle (770) umfasst, wobei das Vollfeld-Swept-Source-OCT-Abbildungssystem (700) so angeordnet ist, dass es eine Reaktion einer Netzhaut eines Auges auf einen von der Lichtquelle (770) erzeugten Lichtreiz misst.

15. Computerimplementiertes Verfahren zur Verarbeitung komplexer volumetrischer OCT-Daten eines Abbildungsziels (130), die entweder durch das Fourier-Domänen-OCT-Abbildungssystem (100) mit Linienabtastung nach Anspruch 1 oder das Vollfeld-Fourier-Domänen-OCT-Bildgebungssystem (700) nach Anspruch 13 erzeugt werden, wobei das Verfahren umfasst:
Erfassen (S10) der komplexen volumetrischen OCT-Daten (170) des Abbildungsziels (130), wobei die komplexen volumetrischen OCT-Daten (170) eine optische Aberration enthalten; und
Erzeugen (S20) korrigierter komplexer volumetrischer OCT-Daten durch Ausführen eines Korrekturalgorithmus, der in den komplexen volumetrischen OCT-Daten kodierte Phaseninformation verwendet, um die komplexen volumetrischen OCT-Daten zu korrigieren, so dass die korrigierten komplexen volumetrischen OCT-Daten (180) weniger von der optischen Aberration aufweisen als die komplexen volumetrischen OCT-Daten (170).

## Revendications

1. Un système d'imagerie par tomographie en cohérence optique, TCO, de domaine de Fourier à champ linéaire (100), comprenant :
une source d'éclairage de champ linéaire (110) agencée pour générer une ligne de lumière (LL) ;
un système de balayage (120) comprenant un élément de balayage (121) et un miroir incurvé (122) ayant un premier point focal (F_{P1}) et un deuxième point focal conjugué (F_{P2}), l'élément de balayage (121) étant situé au premier point focal (F_{P1}) et agencé pour effectuer un balayage d'une cible d'imagerie (130) via le deuxième point focal (F_{P2}), en balayant au moins un segment de la ligne de lumière (LL) à travers la cible d'imagerie (130) via le miroir incurvé (122), l'élément de balayage (121) étant en outre agencé pour recevoir, via le miroir incurvé (122), de la lumière qui a été diffusée par la cible d'imagerie (130) pendant le balayage et aberrée par le miroir incurvé (122) pour former une ligne de lumière aberrée (L_{A}) comprenant au moins une défocalisation ou une distorsion ;
un photodétecteur (140) comprenant un réseau d'éléments photodétecteurs (141) ;
un interféromètre (150) agencé pour recevoir au moins un segment de la ligne de lumière aberrée (L_{A}) via l'élément de balayage (121), générer une ligne d'interférence de lumière (L_{I}) résultant d'une interférence entre une lumière de référence (L_{R}) et le au moins un segment de la ligne de lumière aberrée (L_{A}) reçue via l'élément de balayage (121), et projeter la ligne d'interférence de lumière (L_{I}) sur le réseau d'éléments photodétecteurs (141),
dans lequel le photodétecteur (140) est agencé pour détecter la ligne d'interférence de lumière (L_{I}) projetée sur le réseau d'éléments photodétecteurs (141) pendant le balayage, et générer un signal de détection (S_{d}) basé sur la ligne d'interférence de lumière détectée (L_{I}) ; et
un matériel de traitement de données TCO (160) agencé pour :
générer des données TCO volumétriques complexes (170) de la cible d'imagerie (130) sur la base du signal de détection (S_{d}), dans lequel les données TCO volumétriques complexes (170) comportent une aberration optique ; et
générer des données TCO volumétriques complexes corrigées (180) en exécutant un algorithme de correction (161) qui utilise des informations de phase codées dans les données TCO volumétriques complexes (170) pour corriger les données TCO volumétriques complexes (170), de telle sorte que les données TCO volumétriques complexes corrigées (180) présentent moins d'aberration optique que les données TCO volumétriques complexes (170).

2. Le système d'imagerie TCO de domaine de Fourier à champ linéaire (100) selon la revendication 1, dans lequel
le système d'imagerie TCO de domaine de Fourier à champ linéaire est un système d'imagerie TCO de source balayée de champ linéaire,
la source d'éclairage de champ linéaire (110) est une source d'éclairage de champ linéaire balayée agencée pour générer la ligne de lumière, et
le réseau d'éléments photodétecteurs (141) est un réseau unidimensionnel d'éléments photodétecteurs, et les éléments photodétecteurs du réseau unidimensionnel d'éléments photodétecteurs sont disposés le long d'une longueur d'une projection de la ligne d'interférence de lumière sur le réseau unidimensionnel d'éléments photodétecteurs.

3. Le système d'imagerie TCO de domaine de Fourier à champ linéaire (100) selon la revendication 2, dans lequel les éléments photodétecteurs du réseau unidimensionnel ont une largeur, dans une direction perpendiculaire à une longueur de la projection de la ligne d'interférence de lumière sur le réseau unidimensionnel d'éléments photodétecteurs, qui est plus large qu'une largeur maximale de la projection de la ligne d'interférence de lumière sur le réseau unidimensionnel d'éléments photodétecteurs.

4. Le système d'imagerie TCO de domaine de Fourier à champ linéaire (100) selon la revendication 1, dans lequel
le système d'imagerie TCO de domaine de Fourier à champ linéaire est un système d'imagerie TCO de source balayée de champ linéaire,
la source d'éclairage de champ linéaire (110) est une source d'éclairage de champ linéaire balayée, et
le réseau d'éléments photodétecteurs (141) est un réseau bidimensionnel d'éléments photodétecteurs, et les éléments photodétecteurs du réseau bidimensionnel d'éléments photodétecteurs sont disposés dans une première direction le long d'une longueur d'une projection de la ligne d'interférence de lumière sur le réseau bidimensionnel d'éléments photodétecteurs, et dans une deuxième direction qui est perpendiculaire à la première direction.

5. Le système d'imagerie TCO de domaine de Fourier à champ linéaire (100) selon la revendication 4, dans lequel
une largeur du réseau bidimensionnel d'éléments photodétecteurs, dans la deuxième direction, est plus large qu'une largeur maximale de la projection de la ligne d'interférence de lumière sur le réseau bidimensionnel d'éléments photodétecteurs, et
la projection de la ligne d'interférence de lumière sur le réseau bidimensionnel d'éléments photodétecteurs est couverte dans la deuxième direction par une pluralité des éléments photodétecteurs.

6. Le système d'imagerie TCO de domaine de Fourier à champ linéaire (100) selon la revendication 1, dans lequel
le système d'imagerie TCO de domaine de Fourier à champ linéaire (100) est un système d'imagerie TCO de domaine spectral à champ linéaire,
la source d'éclairage de champ linéaire (110) est une source d'éclairage de champ linéaire à large bande agencée pour générer la ligne de lumière (LL),
le réseau d'éléments photodétecteurs (141) est un réseau bidimensionnel d'éléments photodétecteurs,
et le photodétecteur (140) comprend un spectromètre bidimensionnel comprenant un élément de diffraction et le réseau bidimensionnel d'éléments photodétecteurs, l'élément de diffraction étant agencé pour disperser un contenu spectral de la ligne d'interférence de lumière à travers le réseau bidimensionnel d'éléments photodétecteurs dans une première direction, qui est perpendiculaire à une deuxième direction le long de laquelle s'étend une projection de la ligne d'interférence de lumière sur le réseau unidimensionnel d'éléments photodétecteurs, dans lequel les éléments photodétecteurs du réseau bidimensionnel sont disposés dans la première direction et dans la deuxième direction.

7. Le système d'imagerie TCO de domaine de Fourier à champ linéaire (100) selon l'une quelconque des revendications précédentes, dans lequel
l'élément de balayage (121) est un premier élément de balayage agencé pour balayer le au moins un segment de la ligne de lumière (LL) dans une première direction à travers une surface de la cible d'imagerie (130) de telle sorte que des projections du au moins un segment de la ligne de lumière (LL) sur la surface de la cible d'imagerie (130) pendant le balayage soient déplacées les unes par rapport aux autres le long de la première direction, et
le système de balayage (120) comprend en outre un deuxième élément de balayage, qui est agencé pour réfléchir le au moins un segment de la ligne de lumière (LL) provenant de la source d'éclairage de champ linéaire (110) vers le premier élément de balayage (121), et agencé pour changer, dans une deuxième direction qui est perpendiculaire à la première direction, et dans une troisième direction opposée à la deuxième direction, une position à laquelle le premier élément de balayage (121) doit balayer le au moins un segment de la ligne de lumière (LL) à travers la surface de la cible d'imagerie (130).

8. Le système d'imagerie TCO de domaine de Fourier à champ linéaire (100) selon la revendication 7, dans lequel le premier élément de balayage (121) est agencé pour balayer le au moins un segment de la ligne de lumière (LL) dans la première direction à travers la surface de la cible d'imagerie (130), ou dans une direction opposée à la première direction à travers la surface de la cible d'imagerie (130), à la fois avant et après le changement.

9. Le système d'imagerie TCO de domaine de Fourier à champ linéaire (100) selon l'une quelconque des revendications précédentes, dans lequel le miroir incurvé (122) est un miroir sphéroïdal ayant un axe de symétrie circulaire, et l'élément de balayage (121) est agencé pour effectuer le balayage de la cible d'imagerie (130) en balayant le au moins un segment de la ligne de lumière (LL) à travers la cible d'imagerie (130) via le miroir sphéroïdal de telle sorte que le au moins un segment de la ligne de lumière (LL) incident sur le miroir sphéroïdal se propage dans un plan qui est parallèle à l'axe de symétrie circulaire du miroir sphéroïdal.

10. Le système d'imagerie TCO de domaine de Fourier à champ linéaire (100) selon l'une quelconque des revendications 1 à 8, dans lequel
le miroir incurvé (122) est un miroir sphéroïdal, et
l'élément de balayage (121) est agencé pour effectuer le balayage de la cible d'imagerie (130) en balayant le au moins un segment de la ligne de lumière (LL) à travers la cible d'imagerie (130) via le miroir sphéroïdal de telle sorte qu'une partie du miroir sphéroïdal, sur laquelle le au moins un segment de la ligne de lumière est projeté, présente une symétrie réfléchissante par rapport à un plan parallèle à l'axe de symétrie circulaire et passant par celui-ci.

11. Le système d'imagerie TCO de domaine de Fourier à champ linéaire (100) selon l'une quelconque des revendications précédentes, comprenant en outre un masque (800) ayant une première région (801) et une deuxième région (802) qui entoure la première région (801), dans lequel la première région (801) a une transparence supérieure à celle de la deuxième région (802), le masque (800) étant situé à la fois dans le système d'imagerie TCO de domaine de Fourier à champ linéaire (100) et ayant également la première région (801) formée de manière à permettre à au moins une partie de la ligne d'interférence de lumière (L_{I}) de se propager vers le réseau d'éléments photodétecteurs (141) et à empêcher au moins partiellement de la lumière autre que la ligne d'interférence de lumière (L_{I}) de se propager vers le réseau d'éléments photodétecteurs (1 41).

12. Le système d'imagerie TCO de domaine de Fourier à champ linéaire (100) selon l'une quelconque des revendications précédentes, comprenant en outre une source lumineuse (190), dans lequel le système d'imagerie TCO de domaine de Fourier à champ linéaire (100) est agencé pour mesurer une réponse d'une rétine d'un œil à un stimulus lumineux généré par la source lumineuse (190).

13. Un système d'imagerie par tomographie en cohérence optique, TCO, à balayage de champ complet (700), comprenant :
une source d'éclairage balayée (710) agencée pour générer un faisceau lumineux (L_{B}) dont une longueur d'onde est balayée sur une plage de valeurs pendant l'imagerie par le système d'imagerie TCO à balayage de champ complet (700) ;
un système de transfert (720) comprenant un miroir incurvé (722) ayant un premier point focal (F_{P1}) et un deuxième point focal conjugué (F_{P2}), le système de transfert (720) comprenant en outre un système de focalisation (721) qui est agencé pour focaliser le faisceau lumineux (L_{B})généré par la source d'éclairage balayée (710) au premier point focal (F_{P1}), le miroir incurvé (722) étant agencé pour guider le faisceau lumineux (L_{B}) focalisé au premier point focal (F_{P1}) vers une cible d'imagerie (130) via le deuxième point focal (F_{P2}) de manière à fournir un éclairage plein champ de la cible d'imagerie (130) pendant l'imagerie de la cible d'imagerie (130), le miroir incurvé (722) étant en outre agencé pour recevoir de la lumière qui a été diffusée par la cible d'imagerie (130) pendant l'imagerie de la cible d'imagerie (130) et aberrée par le miroir incurvé (722) pour former un faisceau de lumière aberré (L_{A}) comprenant au moins une défocalisation ou une distorsion ;
un photodétecteur (740) comprenant un réseau bidimensionnel d'éléments photodétecteurs (741) ;
un interféromètre (750) agencé pour recevoir au moins une partie de la lumière qui a été reçue par le miroir incurvé (722) et pour générer une lumière d'interférence (L_{I}) résultant d'une interférence entre une lumière de référence (L_{R}) et la au moins une partie du faisceau de lumière aberré (L_{A}), et pour projeter la lumière d'interférence (L_{I}) sur le réseau bidimensionnel d'éléments photodétecteurs (741),
dans lequel le photodétecteur (740) est agencé pour détecter la lumière d'interférence (L_{I}) projetée sur le réseau bidimensionnel d'éléments photodétecteurs (741) pendant l'imagerie, et générer un signal de détection (S_{d}) basé sur la lumière d'interférence détectée (L_{I}) ; et
un matériel de traitement de données TCO (760) agencé pour :
générer des données TCO volumétriques complexes (170) de la cible d'imagerie (130) sur la base du signal de détection (S_{d}), les données TCO volumétriques complexes (170) comportant une aberration ; et
générer des données TCO volumétriques complexes corrigées (180) en exécutant un algorithme de correction (161) qui utilise des informations de phase codées dans les données TCO volumétriques complexes (170) pour corriger les données TCO volumétriques complexes (170), de telle sorte que les données TCO volumétriques complexes corrigées (180) présentent moins d'aberration que les données TCO volumétriques complexes (170).

14. Le système d'imagerie TCO à balayage de champ complet (700) selon la revendication 13, comprenant en outre une source lumineuse (770), dans lequel le système d'imagerie TCO à balayage de champ complet (700) est agencé pour mesurer une réponse d'une rétine d'un œil à un stimulus lumineux généré par la source lumineuse (770).

15. Un procédé mis en œuvre par ordinateur pour traiter des données TCO volumétriques complexes d'une cible d'imagerie (130) générées soit par le système d'imagerie TCO à champ linéaire de domaine de Fourier (100) de la revendication 1, soit par le système d'imagerie TCO à champ complet de domaine de Fourier (700) de la revendication 13, le procédé comprenant :
l'acquisition (S10) des données TCO volumétriques complexes (170) de la cible d'imagerie (130), les données TCO volumétriques complexes (170) comportant une aberration optique ; et
générer (S20) des données TCO volumétriques complexes corrigées en exécutant un algorithme de correction qui utilise des informations de phase codées dans les données TCO volumétriques complexes pour corriger les données TCO volumétriques complexes, de telle sorte que les données TCO volumétriques complexes corrigées (180) présentent moins d'aberration optique que les données TCO volumétriques complexes (170).
